(19)

**Europäisches Patentamt**

**European Patent Office**

**Office européen des brevets**

(11) **EP 4 592 672 A1**

(12) **EUROPÄISCHE PATENTANMELDUNG**

(43) Veröffentlichungstag:
   **30.07.2025 Patentblatt 2025/31**

(21) Anmeldenummer: **25152014.4**

(22) Anmeldetag: **15.01.2025**

(51) Internationale Patentklassifikation (IPC):
   **G01N 27/413** (2006.01)   **G01N 27/42** (2006.01)
   **G01N 33/497** (2006.01)

(52) Gemeinsame Patentklassifikation (CPC):
   **G01N 27/413; G01N 27/423; G01N 33/4972**

(84) Benannte Vertragsstaaten:
   **AL AT BE BG CH CY CZ DE DK EE ES FI FR GB GR HR HU IE IS IT LI LT LU LV MC ME MK MT NL NO PL PT RO RS SE SI SK SM TR**
   Benannte Erstreckungsstaaten:
   **BA**
   Benannte Validierungsstaaten:
   **GE KH MA MD TN**

(30) Priorität: **23.01.2024 DE 102024101872**

(71) Anmelder: **Dräger Safety AG & Co. KGaA**
   **23560 Lübeck (DE)**

(72) Erfinder:
   • **Baesler, Malte**
     **23558 Lübeck (DE)**
   • **Gimbel, Doreen**
     **23558 Lübeck (DE)**

Bemerkungen:
   Geänderte Patentansprüche gemäss Regel 137(2) EPÜ.

(54) **EINRICHTUNG UND VERFAHREN ZUM ÜBERPRÜFEN EINES ELEKTROCHEMISCHEN SENSORS**

(57)   Die Erfindung betrifft eine Einrichtung und ein Verfahren, um automatisch einen elektrochemischen Sensor zu überprüfen. Der elektrochemische Sensor ist nach Art einer Brennstoffzelle ausgestaltet und umfasst eine Messkammer, zwei Elektroden und einen ionisch leitenden Elektrolyten zwischen den beiden Elektroden. Ein zu detektierendes Zielgas ruft in der Messkammer eine elektrochemische Reaktion hervor. Die Reaktion bewirkt, dass ein elektrischer Strom fließt. Ein Parameter des fließenden elektrischen Stroms korreliert mit der gesuchten Zielgas-Konzentration. Ein Stromstärken-Verlauf [I(t)] wird ermittelt, das ist der zeitliche Verlauf der Stärke des Stroms (I), dessen Fließen durch die elektrochemische Reaktion hervorgerufen wird. Mehrere Parameter [$T_{70}$, $T_{70,35}$, $T_{70,70}$, $T_{norm}$] des Stromstärken-Verlaufs [I(t)] werden ermittelt. Ein Maß für die aktuelle Feuchte des Elektrolyten wird ermittelt. Hierfür werden die ermittelten Parameter [$T_{70}$, $T_{70,35}$, $T_{70,70}$, $T_{norm}$] des Stromstärken-Verlaufs [I(t)] verwendet.

EP 4 592 672 A1

FIG. 4

**Beschreibung**

Die Erfindung betrifft eine Überprüfungs-Einrichtung und ein Überprüfungs-Verfahren, um automatisch einen elektrochemischen Sensor zu überprüfen. Weiterhin betrifft die Erfindung ein Analysegerät und ein Mess-Verfahren, um die Konzentration eines vorgegebenen Zielgases in einer Gasprobe zu ermitteln, wobei das Analysegerät die Überprüfungs-Einrichtung umfasst und wobei das Mess-Verfahren die Schritte des Überprüfungs-Verfahrens umfasst.

Bekannt geworden sind unterschiedliche Analysegeräte mit einem elektrochemischen Sensor, wobei die Analysegeräte dafür verwendet werden, einen Probanden auf Blutalkohol zu untersuchen. Falls sich im Blutkreislauf des Probanden Alkohol befindet, so weist bekanntlich eine vom Probanden abgegebene Atemprobe Atemalkohol auf. Das Analysegerät nimmt einen Teil der abgegebenen Atemprobe als Gasprobe auf, und der elektrochemische Sensor ermittelt den Gehalt von Atemalkohol in der Gasprobe.

Der Erfindung liegt die Aufgabe zugrunde, eine Überprüfungs-Einrichtung und ein Überprüfungs-Verfahren bereitzustellen, um einen elektrochemischen Sensor zu überprüfen und dadurch die Zuverlässigkeit des elektrochemischen Sensors im Vergleich zu bekannten Einrichtungen und Verfahren zu vergrößern. Weiterhin liegt der Erfindung die Aufgabe zugrunde, ein Analysegerät und ein Mess-Verfahren zum Messen der Konzentration eines vorgegebenen Zielgases in einer Gasprobe bereitzustellen, wobei das Analysegerät und das Mess-Verfahren eine größere Zuverlässigkeit als bekannte Analysegeräte und Mess-Verfahren aufweisen sollen.

Die Aufgabe wird durch eine Überprüfungs-Einrichtung mit den Merkmalen des Anspruchs 1 und durch ein Überprüfungs-Verfahren mit den Merkmalen des Anspruchs 9 sowie durch ein Analysegerät mit den Merkmalen des Anspruchs 6 und ein Mess-Verfahren mit den Merkmalen des Anspruchs 11 gelöst. Unteransprüche spezifizieren vorteilhafte Ausgestaltungen. Vorteilhafte Ausgestaltungen der erfindungsgemäßen Überprüfungs-Einrichtung sind, soweit sinnvoll, auch vorteilhafte Ausgestaltungen des erfindungsgemäßen Überprüfungs-Verfahren und umgekehrt. Vorteilhafte Ausgestaltungen des erfindungsgemäßen Analysegeräts sind, soweit sinnvoll, auch vorteilhafte Ausgestaltungen des erfindungsgemäßen Mess-Verfahrens und umgekehrt.

Der zu überprüfende elektrochemische Sensor umfasst eine Messkammer, zwei Elektroden und einen ionisch leitenden Elektrolyten zwischen den beiden Elektroden. Die Messkammer vermag eine Gasprobe aufzunehmen. Die Gasprobe stammt aus einem zu überwachenden räumlichen Bereich. Wenn der räumliche Bereich ein Zielgas mit einer ausreichend großen Konzentration aufweist, so ist das Zielgas in der Regel auch in der Gasprobe vorhanden. Die eine Elektrode ist die Messelektrode, die andere Elektrode die Gegenelektrode. Optional umfasst der elektrochemische Sensor zusätzlich eine Referenzelektrode.

Der Elektrolyt weist eine zeitlich veränderliche Feuchte auf. In einer Ausgestaltung wird unter der Elektrolyt-Feuchte die Stoffzahl oder die Stoffmengen-Konzentration, auch Molarität genannt, verstanden. Die Stoffzahl oder die Stoffmengen-Konzentration bezieht sich auf Teile, die in einer flüssigen Lösung beweglich und daher elektrisch leitfähig sind, bezogen auf das Volumen des Elektrolyten. Je größer die Stoffzahl oder die Stoffmengen-Konzentration sind, desto geringer ist die Elektrolyt-Feuchte. Die Stoffzahl wird beispielsweise in mol] oder [mmol] gemessen, die Stoffmengen-Konzentration beispielsweise in [mol/l]. In der Regel umfasst der Elektrolyt Wasser oder ein anderes Lösungsmittel. Unter der Elektrolyt-Feuchte wird dann die Stoffmengen-Konzentration des Lösungsmittels, gemessen in [mol/l], verstanden.

Der elektrochemische Sensor ist nach Art einer Brennstoffzelle wie folgt ausgestaltet: Ein brennbares Zielgas in der Messkammer ruft eine elektrochemische Reaktion hervor. Die elektrochemische Reaktion bewirkt das Fließen eines elektrischen Stroms zwischen den beiden Elektroden. Eine messbare Detektionsgröße, die vom fließenden elektrischen Strom abhängt, korreliert mit der gesuchten Konzentration des Zielgases in der Messkammer, genau gesagt: mit der Zielgas-Konzentration in einer Gasprobe, die sich in der Messkammer befindet. Die Detektionsgröße ist insbesondere die elektrische Ladung $Q$, die insgesamt im Verlaufe der hervorgerufenen elektrochemischen Reaktion fließt.

Anmerkung: Nachfolgend wird wiederholt die Formulierung verwendet, dass ein Sensor eine physikalische Größe misst, beispielsweise die Zielgas-Konzentration. Hiermit ist Folgendes gemeint: Der Sensor misst direkt die physikalische Größe oder mindestens eine andere Größe, die mit der gesuchten physikalischen Größe korreliert und daher ein Maß für die gesuchte physikalische Größe ist, beispielsweise die elektrische Ladung $Q$ oder eine andere Detektionsgröße. Die Messung liefert mindestens einen Wert für die gesuchte physikalische Größe.

Vorgegeben sind mehrere Verlaufs-Parameter. Jeder vorgegebene Verlaufs-Parameter ist ein Parameter eines zeitlichen Verlaufs einer Stromstärke. Jeder Verlaufs-Parameter charakterisiert den zeitlichen Stromstärken-Verlauf mindestens dann, wenn die Stromstärke gemäß dem zeitlichen Verlauf zunächst größer wird, ein Maximum erreicht und dann wieder kleiner wird. Typischerweise weist die Stärke desjenigen Stroms, der zwischen den Elektroden eines elektrochemischen Sensors fließt, einen solchen zunächst ansteigenden und dann wieder abfallenden Verlauf auf, und zwar mindestens dann, wenn eine Gasprobe in der Messkammer eine ausreichend hohe Konzentration eines brennbaren Zielgases aufweist und die Messkammer während der Messung von der Umgebung ausreichend fluiddicht getrennt ist.

Weiterhin ist eine rechnerauswertbare Feuchte-Funktion vorgegeben. Die Feuchte-Funktion beschreibt die Feuchte des Elektrolyten als Funktion der vorgegebenen Verlaufs-Parameter. Bevorzugt wird die Feuchte-Funktion vorab empirisch mithilfe einer Stichprobe mit mehreren Stichprobenelementen ermittelt, wobei jedes Stichprobenelement für jeden Verlaufs-Parameter jeweils ein Wert und einen tatsächlichen Wert für die Elektrolyt-Feuchte umfasst.

Die erfindungsgemäße Überprüfungs-Einrichtung umfasst ein signalverarbeitendes Steuergerät (control unit). Das Steuergerät vermag einen tatsächlichen Stromstärken-Verlauf (Strommesskurve) zu ermitteln. Der Stromstärken-Verlauf ist der zeitliche Verlauf der Stärke des elektrischen Stroms, dessen Fließen durch die bewirkte elektrochemische Reaktion hervorgerufen wird. Natürlich lässt sich der tatsächliche Stromstärken-Verlauf in der Regel nur näherungsweise ermitteln.

Bevorzugt umfasst die Überprüfungs-Einrichtung einen Stromstärken-Sensor. Dieser Stromstärken-Sensor vermag die Stärke des bewirkten elektrischen Stroms zu messen und ein Signal zu generieren. Das Stromstärken-Signal umfasst eine Information über den zeitlichen Verlauf der gemessenen Stromstärke. Das Steuergerät vermag das generierte Stromstärken-Signal zu empfangen und dadurch die Strommesskurve zu ermitteln.

Das Steuergerät vermag die aktuelle Feuchte des Elektrolyten zwischen den beiden Elektroden des elektrochemischen Sensors wenigstens näherungsweise zu ermitteln. Hierfür verwendet das Steuergerät den ermittelten tatsächlichen Stromstärken-Verlauf. Wie das Steuergerät erfindungsgemäß die Elektrolyt-Feuchte ermittelt, wird nachfolgend dargelegt.

Wie bereits erwähnt, sind mehrere (mindestens zwei) Verlaufs-Parameter vorgegeben. Jeder Verlaufs-Parameter bezieht sich auf einen Stromstärken-Verlauf. Das Steuergerät vermag für jeden Verlaufs-Parameter jeweils zu ermitteln, welchen Wert der Verlaufs-Parameter beim ermittelten tatsächlichen Stromstärken-Verlauf annimmt. Diese Ermittlung liefert mehrere Verlaufs-Parameter-Werte.

Weiterhin ist erfindungsgemäß eine rechnerauswertbare Feuchte-Funktion vorgegeben, wobei die Feuchte-Funktion die Elektrolyt-Feuchte als Funktion der Verlaufs-Parameter beschreibt. Die Feuchte-Funktion ist bevorzugt in einem Datenspeicher der Überprüfungs-Einrichtung abgespeichert, auf den das Steuergerät wenigstens zeitweise Lesezugriff hat, oder ist ein Bestandteil eines Programms, welches das Steuergerät ausführt oder auszuführen vermag. Das Steuergerät vermag die Feuchte-Funktion auf die ermittelten Verlaufs-Parameter-Werte anzuwenden. Diese Anwendung liefert wenigstens näherungsweise die tatsächliche aktuelle Elektrolyt-Feuchte.

Die Erfindung bezieht sich auf einen elektrochemischen Sensor. Ein elektrochemischer Sensor hat im Vergleich zu einem anderen Sensor, der ebenfalls die Konzentration eines brennbaren Zielgases in einer Gasprobe zu messen vermag, insbesondere folgenden Vorteil: Ein Analysegerät mit einem elektrochemischen Sensor verbraucht in der Regel weniger elektrische Energie als ein Analysegerät mit einem andersartigen Sensor, insbesondere weniger als ein Analysegerät mit einem photoelektrischen oder einem oxidierenden Sensor. Dies ist insbesondere dann von Vorteil, wenn das Analysegerät eine eigene Spannungsversorgungseinheit umfasst und überhaupt nicht oder wenigstens nicht dauerhaft mit einem stationären Spannungsversorgungsnetz verbunden ist.

In einer möglichen Anwendung ist das Zielgas Atemalkohol, der in der Atemprobe eines Probanden enthalten sein kann. Atemalkohol absorbiert elektromagnetische Strahlung in einem Wellenlängenbereich um $9,5\ \mu m$. Gerade bei dieser Anwendung ist ein elektrochemischer Sensor häufig in der Herstellung günstiger als ein photoelektrischer Sensor für diesen Wellenlängenbereich.

Ein Zielgas, welches sich als Teil einer Gasprobe in der Messkammer befindet, bewirkt eine elektrochemische Reaktion am elektrochemischen Sensor. Diese elektrochemische Reaktion bewirkt, dass ein elektrischer Strom zwischen den beiden Elektroden fließt. Eine messbare Detektionsgröße eines zeitlichen Verlaufs dieses elektrischen Stroms, insbesondere die elektrische Ladung $Q$, lässt sich als ein Maß für die Konzentration des Zielgases in der Gasprobe verwenden.

Die vom zeitlichen Verlauf abhängende Detektionsgröße, insbesondere die elektrische Ladung $Q$, hängt aber nicht nur von der gesuchten Zielgas-Konzentration ab, sondern auch von der aktuellen Feuchte des Elektrolyten zwischen den beiden Elektroden. Die Elektrolyt-Feuchte kann sich im Laufe der Zeit verändern, und zwar insbesondere dann, wenn das Analysegerät über längere Zeit gelagert wird oder wenn die Messkammer und damit der elektrochemische Sensor des Analysegeräts in einer Fluidverbindung mit der Umgebung steht.

Wenigstens von Zeit zu Zeit muss eine solche Fluidverbindung mit der Umgebung hergestellt werden, damit die Messkammer eine zu untersuchende Gasprobe aufnehmen kann und damit die Messkammer ausgespült werden kann. Außerdem ist eine solche Fluidverbindung häufig auch in einer Situation vorhanden oder kann wenigstens vorhanden sein, in der keine Gasprobe in die Messkammer fließt. Beispielsweise lässt sich eine Fluidverbindung zwischen der Messkammer und der Umgebung mangels eines Verschlusses nicht verschließen, oder die Fluidverbindung wird nicht verschlossen. Oder ein Ventil oder ein sonstiger Verschluss ist nicht vollständig fluiddicht. Außerdem ist es möglich, dass die Messkammer aufgrund von unvermeidlichen Materialtoleranzen und Spalten von der Umgebung nicht ideal fluiddicht getrennt ist.

Falls das Analysegerät längere Zeit in einer Umgebung mit einer relativ geringen Luftfeuchte gelagert wird, so kann Flüssigkeit des Elektrolyten durch unvermeidliche Spalten und Schlitze des Analysegeräts hindurch verdunsten, wodurch die Elektrolyt-Feuchte absinkt. Umgekehrt kann bei einer Lagerung in einer Umgebung mit einer relativ hohen Luftfeuchte die Elektrolyt-Feuchte ansteigen. Dies hat insbesondere folgende Ursache: Oft wird als Elektrolyt eine wässrige Lösung verwendet, beispielsweise eine Lösung von Schwefelsäure in Wasser. Schwefelsäure und andere in Betracht kommende Stoffe im Elektrolyten sind stark hygroskopisch. Wenn die Messkammer in einer Fluidverbindung mit der Umgebung steht, stellt sich zwischen dem Elektrolyten im Inneren eines Geräts mit dem elektrochemischen Sensor und der Umgebung häufig ein Feuchte-Gleichgewicht ein. Dieses Feuchte-Gleichgewicht kann sich auch aufgrund von unvermeidlichen Spalten bei einer längeren Lagerung einstellen. Der Vorgang, dass sich ein Feuchte-Gleichgewicht einstellt, führt oft zu einer relativ großen Volumenänderung des Elektrolyten.

In internen Versuchen haben die Erfinder eine wässrige Lösung von Schwefelsäure als dem Elektrolyten verwendet und bei einer Umgebungstemperatur von 20 °C folgenden Zusammenhang zwischen der Feuchte in der Umgebung und der Schwefelsäure-Konzentration festgestellt:

| Umgebungs-Feuchte in [%] | Schwefelsäure-Konzentration in [mol/l] |
|---|---|
| 10 | 12 |
| 70 | 4 |
| 90 | 2 |

Entsprechend ist die Konzentration des Lösungsmittels Wasser und damit die Elektrolyt-Feuchte umso größer, je größer die Umgebungs-Feuchte ist.

Gemäß einer sinnvollen Definition ist die Elektrolyt-Feuchte die WasserKonzentration im Elektrolyten. Gemäß dieser Definition ist die Elektrolyt-Feuchte umso niedriger, je niedriger die Umgebungs-Feuchte ist.

Die Erfindung ermöglicht es, wenigstens näherungsweise die aktuelle Elektrolyt-Feuchte zu ermitteln. Die Kenntnis der aktuellen Elektrolyt-Feuchte ermöglicht in vielen Fällen insbesondere folgende Schritte:

- Der Einfluss der Elektrolyt-Feuchte auf ein Messergebnis des Sensors lässt sich wenigstens näherungsweise rechnerisch kompensieren. Insbesondere lässt sich der Einfluss der Elektrolyt-Feuchte auf die elektrische Ladung Q oder auf die sonstige Detektionsgröße, die mit der gesuchten Zielgas-Konzentration korreliert, rechnerisch kompensieren. Dadurch steigen die Zuverlässigkeit und / oder die Messgenauigkeit des elektrochemischen Sensors verglichen mit einer Ausgestaltung, bei der die veränderliche Elektrolyt-Feuchte nicht berücksichtigt wird.

- Die Kenntnis der Elektrolyt-Feuchte und insbesondere die Kenntnis von deren Verlauf ermöglicht es, den Elektrolyten automatisch zu überprüfen. Falls die Elektrolyt-Feuchte ausreichend lange außerhalb eines vorgegebenen Soll-Wertebereichs liegt, so lässt sich eine Meldung generieren, dass der elektrochemische Sensor überprüft werden muss und / oder dass Elektrolyt ersetzt oder ergänzt werden muss.

Die Erfindung erfordert in der Regel keinen zusätzlichen Sensor, um die Elektrolyt-Feuchte zu ermitteln. Ein Sensor für die Stärke des Stroms, der zwischen den beiden Elektroden fließt, ist in der Regel ohnehin vorhanden. Ein solcher Sensor wird nämlich in der Regel benötigt, um die Zielgas-Konzentration zu ermitteln. Häufig lässt sich die Erfindung ausschließlich dadurch realisieren, dass eine Software, die von einem Steuergerät angewendet wird, entsprechend verändert wird.

Erfindungsgemäß wird eine Feuchte-Funktion in rechnerauswertbarer Form vorgegeben. Die Feuchte-Funktion beschreibt die Elektrolyt-Feuchte als Funktion der vorgegebenen Verlaufs-Parameter. Bevorzugt wird die Feuchte-Funktion vorab empirisch ermittelt. Um die Feuchte-Funktion zu ermitteln, wird eine Stichprobe mit mehreren Stichprobenelementen generiert und ausgewertet. Jedes Stichprobenelement umfasst in einer Realisierungsform einerseits einen Wert für die tatsächliche Elektrolyt-Feuchte und einen Wert für eine Zielgas-Konzentration und andererseits den tatsächlichen Stromstärken-Verlauf, den der elektrochemische Sensor bei dieser Elektrolyt-Feuchte und dieser Zielgas-Konzentration liefert. In einer anderen Realisierungsform umfasst jedes Stichprobenelement einerseits jeweils ein Wert für die Elektrolyt-Feuchte und die Zielgas-Konzentration und andererseits für jeden vorgegebenen Verlaufs-Parameter jeweils den Wert, den ein Stromstärken-Verlauf annimmt, wobei dieser Stromstärken-Verlauf bei dieser Elektrolyt-Feuchte und dieser Zielgas-Konzentration ermittelt wurde.

Der tatsächliche Stromstärken-Verlauf wird von der Elektrolyt-Feuchte sowie in der Regel von weiteren Faktoren beeinflusst, insbesondere von der Umgebungstemperatur. Die Elektrolyt-Feuchte wird erfindungsgemäß aber nicht direkt in Abhängigkeit vom Stromstärken-Verlauf ermittelt, sondern in Abhängigkeit von den Verlaufs-Parameter-Werten und der Feuchte-Funktion . Daher hat in vielen Fällen die Umgebungstemperatur einen geringeren Einfluss auf die Elektrolyt-Feuchte, als wenn direkt der tatsächlich Stromstärken-Verlauf verwendet werden würde.

Möglich ist, dass die erfindungsgemäße Überprüfungs-Einrichtung einen Temperatursensor umfasst, der die Umgebungstemperatur zu messen vermag, oder dazu ausgestaltet ist, von einem räumlich entfernten Temperatursensor ein Signal zu empfangen, wobei das empfangene Signal eine Information über eine gemessene Temperatur in der Umgebung des elektrochemischen Sensors umfasst.

Die Erfinder haben aber in internen Versuchen festgestellt, dass die tatsächliche Elektrolyt-Feuchte gut mit einer geeigneten Kombination von Werten der Verlaufs-Parametern korreliert. Eine geeignete Wahl der Feuchte-Funktion vermag daher den Einfluss der Umgebungstemperatur sowie von weiteren Einflussfaktoren zu eliminieren oder wenigstens deutlich zu reduzieren. Daher ist es nicht zwingend erforderlich, die Umgebungstemperatur oder weitere möglicherweise beeinflussende Größen zu messen, um die Elektrolyt-Feuchte zu ermitteln. Daher ermöglichen die erfindungsgemäße Verlaufs-Parameter es, in Verbindung mit der Feuchte-Funktion die Elektrolyt-Feuchte relativ gut zu ermitteln. In der Regel weicht die erfindungsgemäß ermittelte Elektrolyt-Feuchte dennoch von der tatsächlichen Elektrolyt-Feuchte ab.

Die Erfindung lässt sich auch in Verbindung mit einer Ausgestaltung verwenden, bei der die Umgebungstemperatur gemessen wird und die Feuchte-Funktion zusätzlich von der Umgebungstemperatur abhängt.

In der Regel lässt sich die aktuelle Feuchte eines Elektrolyten in einem Analysegerät nur mit einem hohen Aufwand direkt messen. Erfindungsgemäß ermittelt das Steuergerät die aktuelle Feuchte des Elektrolyten indirekt. Für diese Ermittlung verwendet das Steuergerät mehrere Verlaufs-Parameter des ermittelten tatsächlichen Stromstärken-Verlaufs. Ausgestaltungen der Erfindung legen unterschiedliche mögliche Verlaufs-Parameter fest, welche sich verwenden lassen, um die Elektrolyt-Feuchte zu ermitteln.

In einer Ausgestaltung ist der oder mindestens ein Verlaufs-Parameter die Zeitspanne zwischen den folgenden beiden Zeitpunkten:

- dem Beginn der Messung (t=0) und
- entweder dem frühesten oder dem spätesten Zeitpunkt, an dem der Stromstärken-Verlauf gleich $x_1$% der maximalen Stromstärke wird.

Der oder ein Verlaufs-Parameter kann auch die Zeitspanne zwischen den folgenden Zeitpunkten sein:

- entweder dem frühesten oder dem spätesten Zeitpunkt, an dem der Stromstärken-Verlauf gleich $x_1$% der maximalen Stromstärke wird, und
- entweder dem frühesten oder dem spätesten Zeitpunkt, an dem der Stromstärken-Verlauf gleich $x_2$% der maximalen Stromstärke wird.

Hierbei sind $x_1$ und $x_2$ zwei Prozentwerte. Die beiden Prozentwerte können miteinander übereinstimmen oder voneinander verschieden sein. Falls an den beiden Zeitpunkten die Stromstärke erstmals $x_1$% bzw. $x_2$% oder letztmals $x_1$% bzw. $x_2$% ist, sind diese beiden Prozentwerte $x_1$% bzw. $x_2$% natürlich voneinander verschieden.

In einer anderen Ausgestaltung ist der oder mindestens ein Verlaufs-Parameter die elektrische Ladung, die insgesamt zwischen den folgenden beiden Zeitpunkten fließt:

- dem Beginn der Messung (t=0) und
- entweder dem frühesten oder dem spätesten Zeitpunkt, an dem der Stromstärken-Verlauf gleich $x_1$% der maximalen Stromstärke wird.

Der oder mindestens ein Verlaufs-Parameter kann auch die elektrische Ladung Q sein, die insgesamt zwischen den folgenden beiden Zeitpunkten fließt:

- entweder dem frühesten oder dem spätesten Zeitpunkt, an dem der Stromstärken-Verlauf gleich $x_1$% der maximalen Stromstärke wird, und
- entweder dem frühesten oder dem spätesten Zeitpunkt, an dem der Stromstärken-Verlauf gleich $x_2$% der maximalen Stromstärke wird.

Die maximale Stromstärke ist der maximale Wert des tatsächlichen Stromstärken-Verlaufs.

Diese beiden Verlaufs-Parameter werden bevorzugt als Fläche unter der Kurve, die den Stromstärken-Verlauf beschreibt, zwischen diesen beiden Zeitpunkten berechnet.

In einer weiteren Ausgestaltung ist der oder mindestens ein Verlaufs-Parameter eine Sekante, insbesondere die Steigung der Sekante, zwischen zwei Punkten auf der Kurve, die den Stromstärken-Verlauf beschreibt. Der jeweilige x-Wert dieser beiden Punkte ist ein bestimmter Zeitpunkt, insbesondere der Beginn der Messung oder der früheste oder auch der späteste Zeitpunkt, an dem der Stromstärken-Verlauf gleich $x_1$% bzw. $x_2$% der maximalen Stromstärke wird. Der jeweilige y-Wert dieser beiden Punkte ist die zugehörige Stromstärke an diesem Zeitpunkt. Ein Sonderfall einer Steigung einer Sekante ist die Steigung der Kurve in einem Punkt.

In einer weiteren Ausgestaltung ist der oder ein Verlaufs-Parameter derjenige Zeitpunkt, an dem die Kurve, die den Stromstärken-Verlauf beschreibt, einen Wendepunkt aufweist.

Erfindungsgemäß ermittelt das Steuergerät die aktuelle Elektrolyt-Feuchte unter Verwendung von mehreren Verlaufs-Parametern (genauer gesagt: von mehreren Verlaufs-Parameter-Werten) des Stromstärken-Verlaufs, wobei verschiedene mögliche Verlaufs-Parameter weiter oben beschrieben wurden. Erfindungsgemäß wendet das Steuergerät die vorgegebene rechnerauswertbare Feuchte-Funktion auf die ermittelten Werte der verwendeten Verlaufs-Parameter an, um die Elektrolyt-Feuchte zu ermitteln.

In einer bevorzugten Ausgestaltung ist die vorgegebene Feuchte-Funktion ein Quotient mit einem Zähler und einem Nenner. Der Zähler ist eine Zähler-Feuchte-Funktion, der Nenner eine Nenner-Feuchte-Funktion. Sowohl im Zähler als auch im Nenner tritt jeweils mindestens ein Verlaufs-Parameter auf. In einer Realisierungsform ist die Zähler-Feuchte-Funktion ein gewichtetes Mittel oder ein Median über mindestens zwei Verlaufs-Parameter, optional auch die Nenner-Feuchte-Funktion.

Die Erfinder haben in internen Versuchen festgestellt, dass bei Verwendung einer Feuchte-Funktion in Form eines derartigen Quotienten der Einfluss von weiteren Faktoren, insbesondere der Umgebungstemperatur, auf die Feuchte-Funktion relativ gut kompensiert wird. Eine Folge: Die erfindungsgemäß ermittelte Elektrolyt-Feuchte hängt relativ wenig von weiteren Einflussfaktoren ab. Falls also die Feuchte-Funktion die Form eines solchen Quotienten hat, so ist es in vielen Fällen nicht erforderlich, die Umgebungstemperatur zu messen und die Feuchte-Funktion zusätzlich von der gemessenen Umgebungstemperatur abhängig zu machen. Diese Ausgestaltung spart daher in vielen Fällen die Notwendigkeit ein, einen Sensor für die Umgebungstemperatur verwenden zu müssen, um die Elektrolyt-Feuchte zu ermitteln.

In der Regel hat der tatsächliche Stromstärken-Verlauf die folgende Form: Die Stromstärke steigt bis zu einem Maximum (im Folgenden: maximale Stromstärke) an und fällt dann wieder ab. In einer Realisierungsform ist der oder ein Verlaufs-Parameter, der in der Nenner-Feuchte-Funktion auftritt, die Zeitspanne zwischen den folgenden beiden Zeitpunkten:

- dem frühesten Zeitpunkt, an dem der Stromstärken-Verlauf gleich $x_1$% der maximalen Stromstärke wird, und
- dem spätesten Zeitpunkt, an dem der Stromstärken-Verlauf gleich $x_2$% der maximalen Stromstärke wird.

$x_1$ und $x_2$ sind zwei Prozentwerte. Diese beiden Prozentwerte $x_1$ und $x_2$ können übereinstimmen oder voneinander verschieden sein. Bevorzugt liegen sowohl $x_1$ als auch $x_2$ zwischen 10% und 30%, besonders bevorzugt zwischen 15% und 25%.

Bei dieser Ausgestaltung wird also über die zeitliche Ausdehnung des tatsächlichen Stromstärken-Verlaufs normiert. Diese Realisierungsform kompensiert den Einfluss der Umgebungstemperatur auf die Feuchte-Funktion und damit auf die erfindungsgemäß ermittelte Elektrolyt-Feuchte besonders gut.

In einer Realisierungsform legt die Feuchte-Funktion fest, dass als Elektrolyt-Feuchte ein gewichtetes Mittel oder ein Median über die verwendeten Verlaufs-Parameter verwendet wird. In einer anderen Ausgestaltung legt die Feuchte-Funktion fest, dass als Elektrolyt-Feuchte ein Quotient verwendet wird. Der Zähler dieses Quotienten ist ein Verlaufs-Parameter oder ein gewichtetes Mittel oder ein Median mehrerer Verlaufs-Parameter. Der Nenner ist ebenfalls ein Verlaufs-Parameter oder ein gewichtetes Mittel oder ein Median mehrerer Verlaufs-Parameter.

Das oder jedes gerade beschriebene gewichtete Mittel wird unter Verwendung von vorgegebenen Gewichtsfaktoren berechnet. Bevorzugt werden diese Gewichtsfaktoren vorab empirisch ermittelt, wobei eine Stichprobe erzeugt und verwendet wird. Jedes Stichprobenelement umfasst jeweils einen gemessenen oder vorgegebenen Wert für die Elektrolyt-Feuchte, einen gemessenen oder vorgegebenen Wert für die Zielgas-Konzentration und den bei diesen beiden Werten resultierenden Stromstärken-Verlauf.

In einer Ausgestaltung vermag das Steuergerät ein Signal zu empfangen, welches eine Information über eine gemessene Temperatur in der Umgebung des elektrochemischen Sensors umfasst. Diese Umgebungstemperatur wird von einem Temperatursensor gemessen. Der Temperatursensor kann ein Bestandteil der erfindungsgemäßen Überprüfungs-Einrichtung sein. Die vorgegebene Feuchte-Funktion hängt von mindestens einem Verlaufs-Parameter und zusätzlich von der Umgebungstemperatur ab.

Die Erfindung betrifft weiterhin ein Analysegerät und ein Mess-Verfahren, welche die Konzentration eines vorgegebenen Zielgases in einer Gasprobe zu messen vermögen. Das Analysegerät umfasst eine Messkammer. Die Messkammer vermag die Gasprobe aufzunehmen. Das Analysegerät umfasst weiterhin einen elektrochemischen Sensor sowie eine erfindungsgemäße Überprüfungs-Einrichtung. Der elektrochemische Sensor ist so aufgebaut, wie dies oben mit Bezug auf die Überprüfungs-Einrichtung beschrieben wurde. Das Mess-Verfahren wird unter Verwendung eines solchen Analysegeräts durchgeführt. Bei der Durchführung des Mess-Verfahrens werden die Schritte des erfindungsgemäßen Überprüfungs-Verfahrens durchgeführt.

Das Analysegerät vermag sich also dank der Überprüfungs-Einrichtung selbst zu überprüfen. Möglich, aber dank der Erfindung nicht erforderlich ist, ein externes Überprüfungsgerät zu verwenden. In vielen Fällen lässt sich dadurch eine zu geringe oder auch eine zu große Elektrolyt-Feuchte des Analysegerätes relativ schnell feststellen. Möglich ist auch, dass eine externe Überprüfungs-Einrichtung erfindungsgemäß die Elektrolyt-Feuchte ermittelt und die ermittelte Elektrolyt-Feuchte an das Analysegerät übermittelt und vom Analysegerät verwendet wird.

Die erfindungsgemäßen Schritte, durch welche die aktuelle Elektrolyt-Feuchte ermittelt wird, werden bevorzugt dadurch ausgelöst, dass folgendes Ereignis detektiert wird: Die gemessene Zielgas-Konzentration liegt außerhalb eines vorgegebenen Konzentrations-Wertebereichs, insbesondere oberhalb einer vorgegebenen unteren Konzentrations-Schranke. Beispielsweise wird die Elektrolyt-Feuchte jedes Mal, wenn die Zielgas-Konzentration oberhalb der Konzentrations-Schranke liegt, erneut ermittelt oder auch jedes n-te Mal, wobei n eine vorgegebene Zahl größer oder gleich 2 ist. Möglich ist auch, die aktuelle Elektrolyt-Feuchte nach jedem n-ten Einsatz des Analysegeräts zu ermitteln, wobei n >= 1 eine vorgegebene Anzahl ist.

Ausgestaltungen der erfindungsgemäßen Überprüfungs-Vorrichtung sind auch Ausgestaltungen des erfindungsgemäßen Analysegeräts. Ausgestaltungen des erfindungsgemäßen Überprüfungs-Verfahrens sind auch Ausgestaltungen des erfindungsgemäßen Mess-Verfahrens.

In einer Ausgestaltung ist in rechnerauswertbarer Form eine Konzentrations-Funktion vorgegeben. Die Konzentrations-Funktion beschreibt die Zielgas-Konzentration als Funktion einerseits der Detektionsgröße und andererseits der Elektrolyt-Feuchte, optional zusätzlich als Funktion der Umgebungstemperatur. Das erfindungsgemäße Analysegerät vermag die Zielgas-Konzentration wie folgt zu messen: Das Analysegerät wendet die Konzentrations-Funktion auf die gemessene Detektionsgröße und auf die ermittelte Elektrolyt-Feuchte an, optional zusätzlich auf die gemessene Umgebungstemperatur. In vielen Fällen ist die folgende Annahme gerechtfertigt und lässt sich beim Aufstellen der Konzentrations-Funktion anwenden: Bei konstanter tatsächlicher Zielgas-Konzentration und optional bei konstanter Umgebungstemperatur hängt die Detektionsgröße linear von der Elektrolyt-Feuchte ab, und zwar bevorzugt so, dass die Detektionsgröße umso kleiner ist, je kleiner die Elektrolyt-Feuchte ist. Möglich ist auch, vorab empirisch einen funktionalen Zusammenhang zu ermitteln, wobei der ermittelte funktionale Zusammenhang die Detektionsgröße als Funktion der Zielgas-Konzentration und der Elektrolyt-Feuchte und optional der Umgebungstemperatur beschreibt.

Erfindungsgemäß wird vorab eine Feuchte-Funktion aufgestellt und während eines Einsatzes der Überprüfungs-Einrichtung verwendet. Im vorigen Absatz wurde eine Ausgestaltung beschrieben, bei der vorab eine Konzentrations-Funktion aufgestellt wird. Diese Konzentrations-Funktion wird bei einem Einsatz des Analysegeräts verwendet. In der Regel wird für beide Anwendungen jeweils eine Stichprobe benötigt. Beispielsweise wird mithilfe der Stichprobe für mindestens einen Modellparameter der Feuchte-Funktion und / oder für mindestens einen Modellparameter der Konzentrations-Funktion ein Wert ermittelt. Oder ein lernendes Verfahren wird angewendet, um die Feuchte-Funktion und / oder die Konzentrations-Funktion aufzustellen.

Die nachfolgend beschriebene Ausgestaltung erspart die Notwendigkeit, hierfür die tatsächliche Elektrolyt-Feuchte zu messen. Vielmehr wird folgende Tatsache ausgenutzt: Wenigstens unter Laborbedingungen führt eine bestimmte Umgebungs-Feuchte zu einer bestimmten Elektrolyt-Feuchte, vorausgesetzt zwischen dem elektrochemischen Sensor mit dem Elektrolyten und der Umgebung ist ausreichend lange eine ausreichende Fluidverbindung hergestellt, und die Umgebungs-Feuchte bleibt konstant. Unter Laborbedingungen lässt sich in der Regel ausreichend zuverlässig eine bestimmte bekannte Umgebungs-Feuchte herstellen. Je größer die Umgebungs-Feuchte ist, desto größer ist auch die Elektrolyt-Feuchte. Andere Einflussgrößen, insbesondere die Temperatur, nehmen einen deutlich geringeren Einfluss auf die Elektrolyt-Feuchte.

Vorgegeben wird ein Wertebereich von a bis b, beispielsweise von 0 bis 1 oder von -1 bis +1. Die Elektrolyt-Feuchte, die sich bei der kleinstmöglichen Umgebungs-Feuchte von beispielsweise 0 % einstellt, wird mit a codiert, die Elektrolyt-Feuchte bei der größtmöglichen Umgebungs-Feuchte von beispielsweise 100 % mit b. Jede Elektrolyt-Feuchte, die aus einer Umgebungs-Feuchte zwischen dem kleinstmöglichen dem größtmöglichen Wert resultiert, wird mit einem Wert zwischen a und b codiert. Welchen tatsächliche Wert die Elektrolyt-Feuchte bei einer bestimmten Umgebungs-Feuchte tatsächlich annimmt, braucht nicht gemessen zu werden.

Gemäß dieser Ausgestaltung liefert die erfindungsgemäße Feuchte-Funktion einen Wert aus dem Intervall von a bis b für die gesuchte Elektrolyt-Feuchte. In der Konzentrations-Funktion tritt nicht die tatsächliche Elektrolyt-Feuchte auf, sondern die codierte Elektrolyt-Feuchte. Bei einem Einsatz des Analysegeräts wird die codierte Elektrolyt-Feuchte, also ein Wert aus dem Intervall von a bis b, erfindungsgemäß ermittelt und in die Konzentrations-Funktion eingesetzt.

Das Analysegerät kann als ein tragbares Gerät ausgestaltet sein, welches dazu ausgestaltet ist, von einem Menschen in einer Hand gehalten oder an der Schutzbekleidung eines Menschen befestigt zu werden. Bei dieser Ausgestaltung hat das Analysegerät bevorzugt eine eigene Spannungsversorgungseinheit. Außerdem hat das Analysegerät bevorzugt eine eigene Ausgabeeinheit, auf der die ermittelte Zielgas-Konzentration und / oder eine Warnung betreffend eine zu große oder zu geringe Zielgas-Konzentration ausgegeben wird, und zwar in mindestens einer von einem Menschen wahrnehmbaren Form. Auf dieser Ausgabeeinheit wird in einer Ausgestaltung auch eine zu geringe oder eine zu große Elektrolyt-Feuchte angezeigt. Die Meldungen werden beispielsweise visuell, akustisch und / oder haptisch (durch Vibrationen) ausgegeben.

Das Analysegerät kann auch als ein stationäres Gerät ausgestaltet sein, welches an einem Ort aufgestellt wird und sich bevorzugt mit einem stationären Spannungsversorgungsnetz verbinden lässt. Bevorzugt umfasst bei dieser Anwendung das Analysegerät eine Kommunikationseinheit, welche eine Nachricht mit einer ermittelten Zielgas-Konzentration an einen räumlich entfernten Empfänger zu übermitteln vermag. Optional wird auch eine Elektrolytfeuchte, die das Analysegerät erfindungsgemäß übermittelt hat, an den Empfänger übermittelt, und zwar mindestens dann, wenn die Elektrolyt-Feuchte zu groß oder zu klein ist.

Das Zielgas ist bevorzugt ein brennbares Zielgas, beispielsweise Atemalkohol oder Methan oder Wasserstoff oder auch ein Narkosemittel. Möglich ist, dass das Analysegerät die summierten Konzentrationen von mehreren Zielgasen zu ermitteln vermag.

Im Folgenden wird die Erfindung anhand eines Ausführungsbeispiels beschrieben. Hierbei zeigt

Figur 1      schematisch ein Analysegerät mit einem elektrochemischen Sensor;

Figur 2      schematisch die Wirkungsweise eines elektrochemischen Sensors;

Figur 3      den jeweiligen zeitlichen Verlauf der Stromstärke bei verschiedenen Umgebungstemperaturen und verschiedenen Elektrolyt-Feuchten;

Figur 4      den zeitlichen Verlauf der Stromstärke sowie einige Verlaufs-Parameter dieses zeitlichen Verlaufs;

Figur 5      den zeitlichen Verlauf von Figur 4 und einige weitere Verlaufs-Parameter.

Figur 6      beispielhafte Messergebnisse.

Die Erfindung lässt sich für ein Analysegerät verwenden, welches die Konzentration mindestens eines Zielgases in einem Gasgemisch misst. In einer Anwendung ist das Gasgemisch eine Atemprobe eines Probanden, und das Zielgas ist Atemalkohol. Festgestellt werden soll, ob im Blutkreislauf des Probanden Alkohol vorhanden ist oder nicht. Alkohol im Blutkreislauf führt bekanntlich zu Atemalkohol in einer Atemprobe. In anderen Anwendungen ist das Gasgemisch die Umgebungsluft, und das Zielgas ist bei einer Anwendung ein brennbares oder auf andere Weise für einen Menschen schädliches Gas und bei einer anderen Anwendung Sauerstoff oder ein Anästhesiemittel.

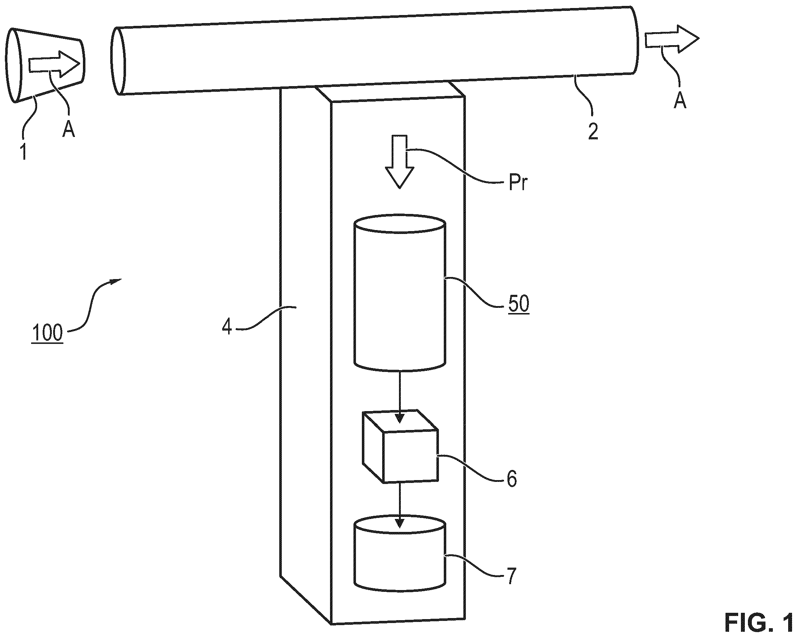

Figur 1 zeigt schematisch ein Analysegerät 100. Das Analysegerät 100 umfasst ein Gehäuse 4, in dem eine Sensor-Anordnung 50, ein signalverarbeitendes Steuergerät (control unit) 6 und ein Datenspeicher 7 angeordnet sind. Die Sensor-Anordnung 50 umfasst einen elektrochemischen Sensor und eine Messkammer. Eine Person kann das Gehäuse 4 in einer Hand halten. Diese Person kann der Proband sein oder eine andere Person, beispielsweise ein Polizist. Auf das Gehäuse 4 ist eine Röhre 2 aufgebracht, bevorzugt lösbar aufgebracht. Ein trichterförmiges Mundstück 1 lässt sich mit der Röhre 2 verbinden.

Die Person hält das Analysegerät 100 so, dass das Mundstück 1 sich vor dem Mund eines Probanden befindet. Der Proband gibt durch Ausatmen eine Atemprobe A in das Mundstück 1 ein. Die Atemprobe A fließt durch die Röhre 2. Ein Teil der Atemprobe A wird aus der Röhre 2 abgezweigt und fließt als eine Messkammer-Probe Pr in die Messkammer. Bevorzugt saugt eine geeignete Fluidfördereinheit (nicht gezeigt) die Messkammer-Probe Pr aus der Atemprobe A an und fördert sie in die Messkammer. Der Rest der Atemprobe A fließt durch die Röhre 2 hindurch wieder in die Umgebung.

Das Analysegerät 100 umfasst also eine Messkammer, welche die zu untersuchende Messkammer-Probe Pr aufnimmt, und einen Sensor, der die Konzentration des Zielgases in der Gasprobe misst. Unterschiedliche Prinzipien, die ein solcher Sensor anwenden kann, sind bekannt geworden. Ein elektrochemischer Sensor, wie er nachfolgend beschrieben wird, hat im Vergleich zu anderen Sensorprinzipien den Vorteil, dass der elektrochemische Sensor weniger elektrische Energie benötigt. Dies ist insbesondere dann von Vorteil, wenn das Analysegerät 100 ohne dauerhafte Verbindung zu einem stationären Spannungsversorgungsnetz verwendet werden sollen und daher eine eigene Spannungsversorgungseinheit umfasst.

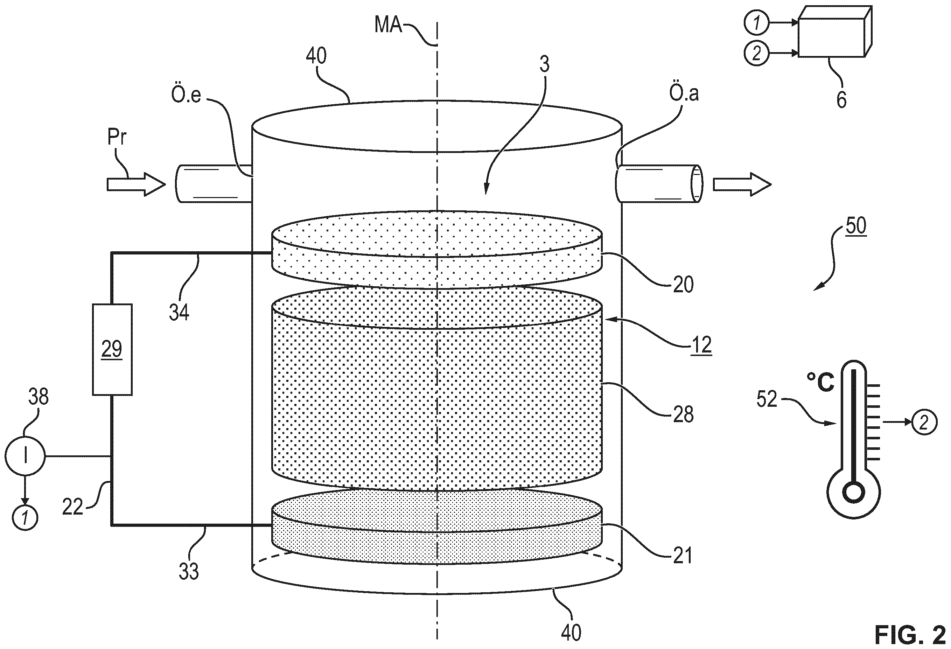

Die Erfindung lässt sich dafür verwenden, einen solchen elektrochemischen Sensor zu überprüfen. Figur 2 zeigt schematisch und beispielhaft die Wirkungsweise eines elektrochemischen Sensors 12, wie er aus dem Stand der Technik bekannt ist. Die Darstellung von Figur 2 ist nicht notwendigerweise maßstabsgerecht.

Dieser elektrochemische Sensor 12 gehört zur Sensor-Anordnung 50 von Figur 1. Der Sensor 12 vermag eine Messkammer-Probe Pr auf ein Zielgas zu analysieren und arbeitet nach dem Prinzip einer Brennstoffzelle mit dem Zielgas als dem Brennstoff. Das Zielgas ist beispielsweise Atemalkohol, das in der Atemprobe A des Probanden enthalten ist, oder ein Aldehyd, das bei der Oxidation von Alkohol entsteht.

Mit dem Bezugszeichen 50 wird in Figur 1 und Figur 2 eine Sensor-Anordnung bezeichnet, welche den eigentlichen elektrochemischen Sensor 12 und eine Wandung 40 für eine Messkammer 3 umfasst. Die Wandung 40 umgibt den Sensor 12 und die Messkammer 3. In der gezeigten Realisierungsform sind sowohl die Wandung 40 als auch der Sensor 12 rotationssymmetrisch zu derselben Mittelachse MA, die in der Zeichenebene von Figur 2 liegt. Selbstverständlich sind auch andere geometrische Formen möglich. Außerdem wird in Figur 1 und Figur 2 schematisch das signalverarbeitende Steuergerät 6 gezeigt.

Die zu analysierende Messkammer-Probe Pr, die im Ausführungsbeispiel von der Atemprobe A stammt, fließt durch eine eintrittsseitige Öffnung Ö.e hindurch in das Innere der Messkammer 3, z.B. indem sie angesaugt wird und / oder in die Messkammer 3 hinein diffundiert. In einer Ausgestaltung fließt die Messkammer-Probe Pr durch eine austrittsseitige Öffnung Ö.a wieder aus der Messkammer 3 heraus. Dank dieser Ausgestaltung kann der Sensor 12 rasch nacheinander mehrere Messkammer-Proben Pr untersuchen. Möglich ist auch, dass keine austrittsseitige Öffnung Ö.a vorhanden ist und die Messkammer-Probe Pr durch die eintrittsseitige Öffnung Ö.e hindurch wieder aus der Messkammer 3 herausfließt.

Der elektrochemische Sensor 12 umfasst

- eine Messelektrode 20, die durch einen Kontaktierungsdraht 34 elektrisch kontaktiert ist,
- eine Gegenelektrode 21, die durch einen Kontaktierungsdraht 33 elektrisch kontaktiert ist,
- einen ionisch leitenden Elektrolyten 28 zwischen den beiden Elektroden 20 und 21,
- einen Verbindungsdraht 22, der die beiden Kontaktierungsdrähte 33 und 34 elektrisch miteinander verbindet und einen elektrischen Messwiderstand 29 umfasst,
- optional eine nicht gezeigte Referenzelektrode und
- einen Stromstärken-Sensor 38, der wiederholt die aktuelle Stärke I des durch den Verbindungsdraht 22 fließenden Stroms misst.

Der Elektrolyt 28 wird bevorzugt von einer Membrane bereitgestellt. Ein solcher elektrochemischer Sensor 12 wird nachfolgend auch als Membran-Elektroden-Elektrolyt-Einheit (MPEE) bezeichnet.

Der Elektrolyt 28 ist ein elektrisch leitendes Medium, beispielsweise mit Wasser verdünnte Schwefelsäure oder Phosphorsäure oder Perchlorsäure. In dem Elektrolyten 28 können sich Ionen als elektrische Ladungsträger bewegen, was die elektrische Leitfähigkeit bewirkt. Bevorzugt stellt eine poröse Membrane den Elektrolyten 28 bereit. Der Elektrolyt 28 stellt eine ionisch leitfähige Verbindung zwischen der Messelektrode 20 und der Gegenelektrode 21 her.

Das Steuergerät 6 empfängt vom Stromstärken-Sensor 38 ein Signal, welches die gemessene Stromstärke I = I(t) beschreibt.

In einer Ausgestaltung misst ein Temperatursensor 52 die Temperatur in einer Umgebung der Sensoranordnung 50. Das Steuergerät 6 empfängt vom Temperatursensor 52 ein Signal, welches die gemessene Umgebungstemperatur beschreibt.

Der Sensor 12 ist so ausgestaltet, dass die Messkammer-Probe Pr nur die Messelektrode 20 erreicht, aber nicht die Gegenelektrode 21. Im gezeigten Beispiel befindet die Messelektrode 20 sich an einer Wand der Messkammer 3, und die Wandung 40 und der Elektrolyt 28 verhindern, dass eine relevante Menge der Messkammer-Probe Pr die Gegenelektrode 21 erreicht.

Die beiden Kontaktierungsdrähte 33 und 34 sind elektrisch leitend und bestehen aus einem Material, welches vom Elektrolyten 28 nicht chemisch angegriffen wird, beispielsweise aus Platin oder Gold. Auch die Elektroden 20 und 21 bestehen aus einem chemisch resistenten Material, beispielsweise ebenfalls aus Platin oder Gold. In vielen Fällen fungiert das chemisch resistente Material der Elektroden 20, 21 zusätzlich als Katalysator für eine chemische Reaktion, die vom Zielgas, hier also vom Atemalkohol, hervorgerufen und zur Messung verwendet wird.

In einer Realisierungsform funktioniert der elektrochemische Sensor 12 nach dem Prinzip einer Brennstoffzelle. Die Messelektrode 20 und der Elektrolyt 28 adsorbieren das Zielgas, hier das Ethanol, in der Messkammer-Probe Pr. Das adsorbierte Ethanol wird dann nach dem Prinzip der Brennstoffzelle oxidiert. Die chemische Reaktion, die zur Messung verwendet wird, umfasst also den Schritt, dass in der Messkammer 3 der Atemalkohol in der Messkammer-Probe Pr oxidiert wird. Idealerweise wird die gesamte Menge des Atemalkohols in der Messkammer-Probe Pr oxidiert.

Als Folge der chemischen Reaktion fließt ein elektrischer Strom I zwischen der Messelektrode 20 und der Gegenelektrode 21 und damit durch den Verbindungsdraht 22 mit dem Messwiderstand 29. Der Stromstärken-Sensor 38 misst die zeitlich veränderliche Stromstärke I. Das Steuergerät 6 leitet die elektrische Ladung Q her, also die gesamte Menge des elektrischen Stroms, der durch den Verbindungsdraht 22 fließt (Prinzip der Coulometrie). In der Regel fließt elektrischer Strom so lange, bis das gesamte elektrochemisch oxidierbare Gas, hier also aller Atemalkohol, das in der Messkammer 3 vorhanden ist, tatsächlich elektrochemisch umgesetzt ist. Bei einem gegebenen Volumen der Messkammer-Probe Pr in der Messkammer 3 ist die gemessene elektrische Ladung Q umso höher, je mehr Atemalkohol die Messkammer-Probe Pr vor der elektrochemischen Umsetzung enthält. Die gemessene elektrische Ladung Q ist daher ein Maß für den Atemalkohol-Gehalt in der Messkammer-Probe Pr und somit für den Gehalt an Alkohol im Blut des Probanden.

Das Steuergerät 6 wendet einen vorgegebenen Zusammenhang an, um aus der gemessenen Ladung Q die gesuchte Konzentration con von Atemalkohol in der Messkammer-Probe Pr herzuleiten:

$$(1) \quad con = F(Q),$$

beispielsweise

$$(2) \quad con = \alpha * Q.$$

Dieser Zusammenhang sowie weitere nachfolgend beschriebene Zusammenhänge werden vorab in einer rechnerausführbaren Form aufgestellt und sind in einer rechnerauswertbaren Form im Datenspeicher 7 abgespeichert. Das Steuergerät 6 hat wenigstens zeitweise Lesezugriff auf den Datenspeicher 7.

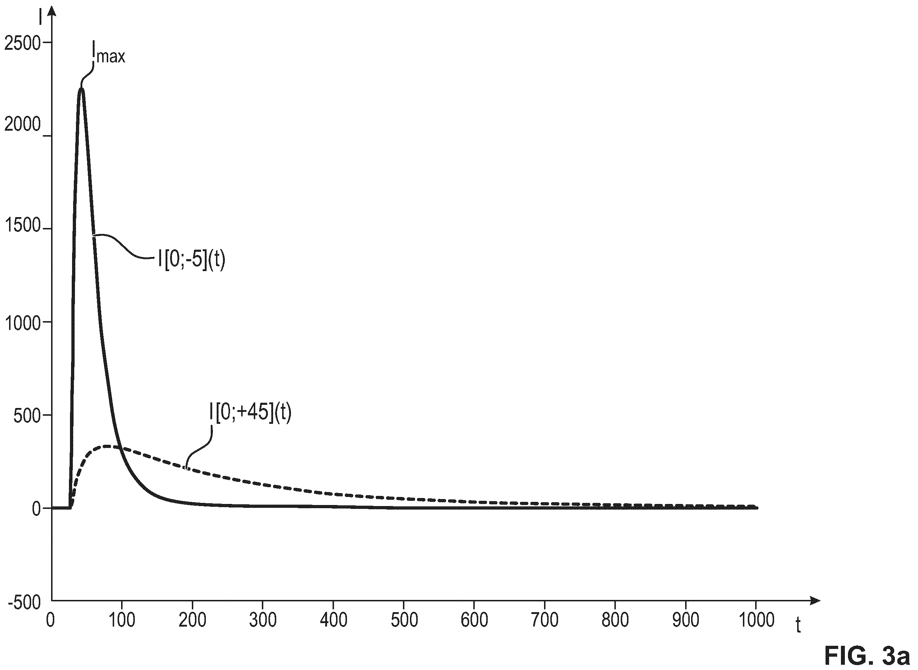

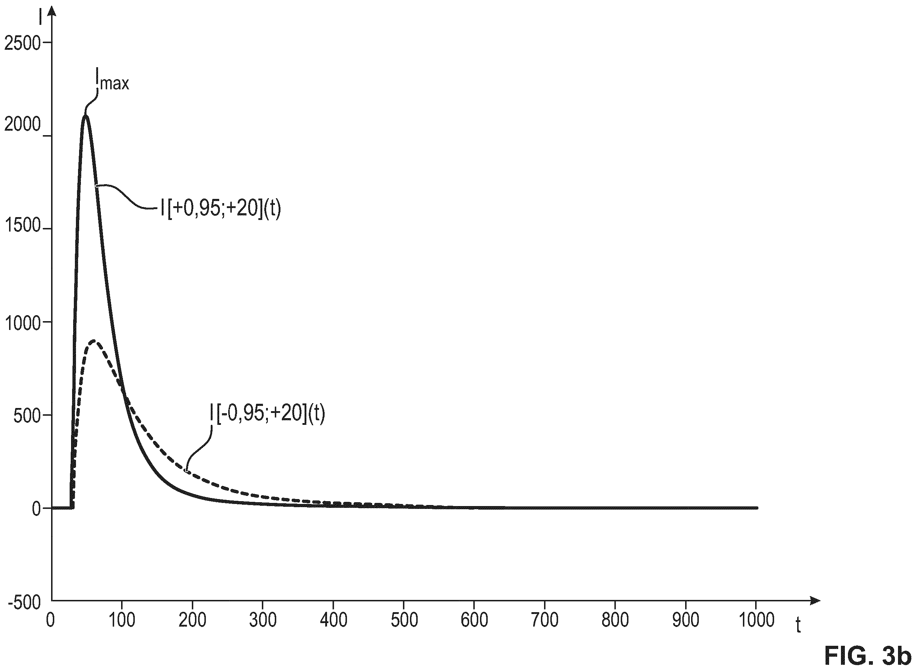

Figur 3 zeigt beispielhaft vier verschiedene Stromstärken-Verläufe und illustriert die Abhängigkeit von der Elektrolyt-Feuchte Ef und von der Umgebungstemperatur. Auf der x-Achse ist die Zeit aufgetragen, auf der y-Achse die jeweilige Stromstärke I. Mit I[Ef;Temp](t) wird der Stromstärken-Verlauf bei einer Elektrolyt-Feuchte Ef und einer Umgebungstemperatur von Temp bezeichnet. Bei allen vier Kurven ist Atemalkohol in der untersuchten Gasprobe mit der gleichen Konzentration vorhanden.

In diesem Beispiel wird die mögliche Elektrolyt-Feuchte Ef mit einem Wert aus dem Wertebereich von -1 (minimale Elektrolyt-Feuchte) bis +1 (maximale Elektrolyt-Feuchte) codiert. Nicht erforderlich ist es, die tatsächliche Elektrolyt-Feuchte zu messen. In Figur 3a nimmt die Elektrolyt-Feuchte Ef den Wert 0 an, also einen mittleren Wert. Die Umgebungstemperatur beträgt -5 °C bzw. +45 °C. Bei einer hohen Umgebungstemperatur wird eine hohe maximale Stromstärke $I_{max}$ erzielt, die Stromstärke nimmt aber sehr schnell ab. Bei beiden Kurven ist die Fläche unter der Kurve etwa gleich, d. h. die gleiche Menge von brennbaren Zielgas wurde oxidiert, und die gleiche elektrische Ladung Q ist geflossen.

In Figur 3b beträgt die Umgebungstemperatur +20 °C. Die Elektrolyt-Feuchte nimmt den Wert von +0,95 bzw. -0,95 an, ist also sehr gering (relativ trockener Elektrolyt) bzw. sehr groß (relativ feuchter Elektrolyt). Bei einer hohen Elektrolyt-Feuchte Ef wird ähnlich wie bei einer hohen Umgebungstemperatur eine hohe maximale Stromstärke $I_{max}$ erzielt, die Stromstärke I nimmt aber schnell wieder ab. Außerdem sind bei einer hohen Elektrolyt-Feuchte Ef die elektrische Ladung Q und damit die Fläche unter der Kurve größer als bei einer geringen Elektrolyt-Feuchte Ef.

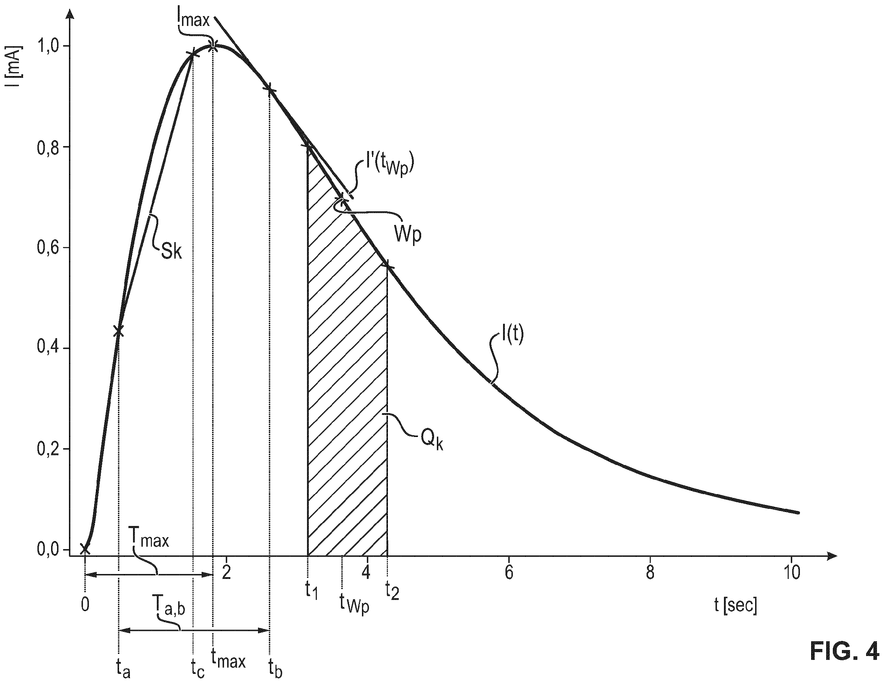

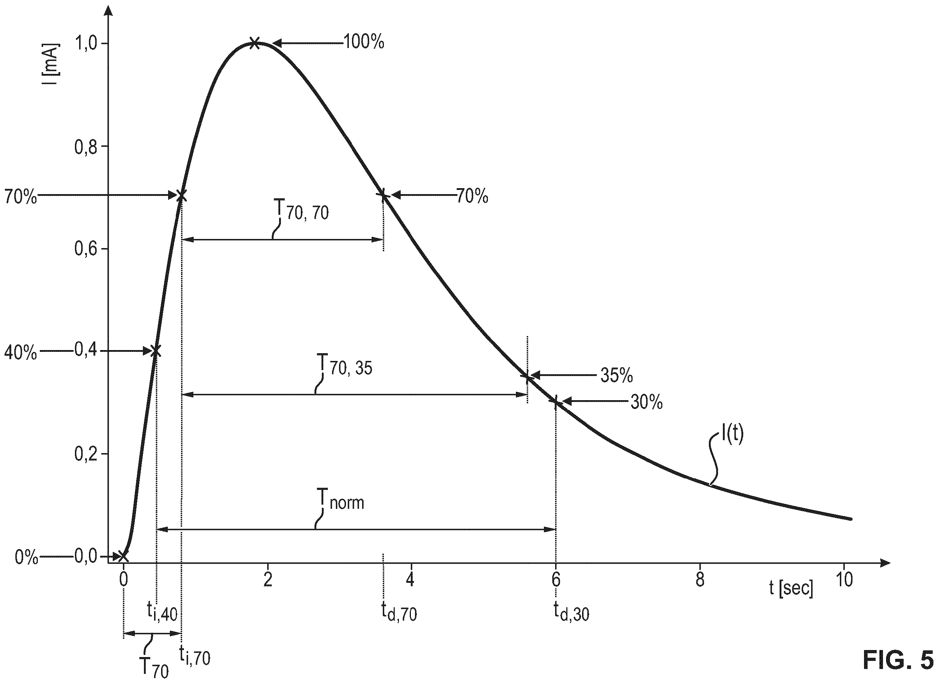

Figur 4 und Figur 5 zeigen einen beispielhaften zeitlichen Verlauf bei der Analyse der Messkammer-Probe Pr. Auf der x-Achse ist die Zeit t eingetragen, auf der y-Achse die zum Zeitpunkt t jeweils gemessene Stromstärke I(t) in [mA]. Figur 4 und Figur 5 zeigen also beispielhaft einen typischen Stromstärken-Verlauf (Strommesskurve) I(t). Dieser Stromstärken-Verlauf I(t) tritt auf, wenn die Messkammer-Probe Pr Atemalkohol umfasst. Die Stromstärke I steigt typischerweise vom Beginn der Messung bis zu einem Maximum $I_{max}$ an und fällt dann wieder ab.

Bekanntlich ist die Fläche unter der Kurve I(t) von t=0 bis zu einem Zeitpunkt t>0 gleich der elektrischen Ladung Q, die bis zum Zeitpunkt T geflossen ist.

Das Steuergerät 6 ermittelt aus dem Stromstärken-Verlauf I(t) die elektrische Ladung Q. Die elektrische Ladung Q fungiert als die Detektionsgröße für den Gehalt an Atemalkohol. Die gemessene elektrische Ladung Q hängt einerseits vom gesuchten Gehalt an Atemalkohol in der Atemprobe A und damit in der Messkammer-Probe Pr ab, andererseits von folgenden Variablen:

- der Elektrolyt-Feuchte Ef,
- der Feuchte der Messkammer-Probe Pr,
- die Temperatur der Elektroden 20 und 21 und
- der Umgebungstemperatur Temp.

In vielen Fällen kann für die Feuchte der Atemprobe A und damit der Messkammer-Probe Pr ein vorgegebener Standardwert verwendet werden. Auch für die Umgebungstemperatur kann häufig ein vorgegebener Standardwert verwendet werden. Möglich ist auch, dass das Steuergerät 6 einen Messwert für die aktuelle Umgebungstemperatur empfängt und verarbeitet.

In einer Ausgestaltung umfasst das Analysegerät 100 einen Sensor, der die Temperatur der Elektroden 20 und 21 misst. Häufig kann ein linearer Einfluss der Elektroden-Temperatur auf die elektrische Ladung Q angenommen werden. In einer anderen Ausgestaltung wird ein Standardwert oder Referenzwert für die Elektrodentemperatur verwendet.

Nachfolgend wird mit $Q_{meas}$ die gemessene und optional um den Einfluss der Feuchte der Messkammer-Probe Pr, der Elektroden-Temperatur und / oder der Umgebungstemperatur korrigierte elektrische Ladung Q bezeichnet.

Wie der Einfluss der Elektrolyt-Feuchte Ef auf die gemessene Ladung $Q_{meas}$ berücksichtigt wird, wird nachfolgend beschrieben. Die Feuchte des Elektrolyten 28 wird von Umgebungsbedingungen beeinflusst, insbesondere von der Umgebungs-Feuchte. Zwangsläufig stehen nämlich die Messkammer 3 und damit der Elektrolyt 28 wenigstens zeitweise in einer Fluidverbindung mit der Umgebung, nämlich wenigstens dann, wenn der Proband eine Atemprobe A eingibt und ein Teil der Atemprobe A als die Messkammer-Probe Pr in die Messkammer 3 fließt. Außerdem sinkt in der Regel die Elektrolyt-Feuchte Ef, wenn das Analysegerät 100 längere Zeit in einer Umgebung mit einer relativ geringen relativen Feuchte gelagert wird.

In der Regel ist bei gleichbleibender Zielgas-Konzentration die gemessene elektrische Ladung $Q_{meas}$ umso größer, je größer die Elektrolyt-Feuchte Ef ist. Daher wendet das Steuergerät 6 eine rechnerauswertbare Konzentrations-Funktion

$$(3) \qquad con = F[Ef](Q_{meas})$$

an, welche den Einfluss der Elektrolyt-Feuchte Ef berücksichtigt.

Bei einer konstanten Konzentration con des Zielgases, hier des Atemalkohols, ist die Ladung $Q_{meas}$ also umso größer, je größer die Elektrolyt-Feuchte Ef ist. Daher gilt

$$(4) \qquad Q_{meas} = G(Ef) * Q_{ref}.$$

Hierbei ist $Q_{ref}$ ein Referenzwert für die elektrische Ladung Q, der unter vorgegebenen Referenzbedingungen auftritt, wie beispielsweise bei einer bestimmten Zielgas-Konzentration, einer Elektrolyt-Feuchte Ef von 50% (0 auf der Skala von -1 bis +1) und einer bestimmten Referenztemperatur. Die Umgebungs-Feuchte kann auf die Elektrolyt-Feuchte Ef einwirken, beeinflusst aber in der Regel nicht direkt in erheblichem Ausmaß die elektrische Ladung $Q_{meas}$. In vielen Fällen ist die Annahme gerechtfertigt, dass bei einer konstanten Konzentration con von Atemalkohol zwischen der elektrischen Ladung Q und der Elektrolyt-Feuchte Ef ein linearer Zusammenhang der Form

$$(5) \qquad G(Ef) = a + b * Ef, \text{ also } Q_{meas} = (a + b * Ef) * Q_{ref,}$$

mit ausreichender Genauigkeit die Abhängigkeit beschreibt.

In Figur 4 und Figur 5 sind unterschiedliche Verlaufs-Parameter des Stromstärken-Verlaufs I(t) dargestellt. In Figur 4 sind u.a. dargestellt:

- die maximale Stromstärke $I_{max}$,
- der Wendepunkt Wp, an dem die Krümmung des Stromstärken-Verlaufs I(t) sich ändert, sowie der Zeitpunkt $t_{wp}$, an dem dieser Wendepunkt Wp auftritt,
- die Zeitspanne $T_{max}$, die zwischen dem Beginn t = 0 der Messung und dem Zeitpunkt $t_{max}$ verstreicht, an dem der Strom mit der maximalen Stromstärke $I_{max}$ fließt,
- ein Teilintegral $Q_k$, das ist die Ladung, die zwischen zwei Zeitpunkten $t_1$ und $t_2$ fließt, also die Fläche unter dem Stromstärken-Verlauf I(t) zwischen diesen beiden Zeitpunkten $t_1$ und $t_2$, wobei beispielsweise der Zeitpunkt $t_{Wp}$ des Wendepunkts Wp zwischen den beiden Zeitpunkten $t_1$ und $t_2$ liegt oder wobei zum Zeitpunkt $t_1$ die Stromstärke erstmals oder letztmals $x_1$% der maximalen Stromstärke beträgt und zum Zeitpunkt $t_2$ erstmals oder letztmals $x_2$%
- die Zeitspanne $T_{a,b}$, die zwischen zwei charakteristischen Zeitpunkten $t_a$ und $t_b$ des Stromstärken-Verlaufs I(t) verstreicht,
- die Veränderung $I'(t_b)$ der Stromstärke I(t) zu einem bestimmten Zeitpunkt $t_b$, also die Steigung des Stromstärken-Verlaufs I(t) zum Zeitpunkt $t_b$, und
- die Steigung der Sekante Sk zwischen zwei charakteristischen Punkten $t_a$ und $t_b$, also $[I(t_c) - I(t_a)] / (t_c - t_a)$.

In Figur 5 sind die Zeitpunkte veranschaulicht, an denen der Stromstärken-Verlauf I(t) 30%, 35%, 40%, 70% und 100% der maximalen Stromstärke $I_{max}$ erreicht. Dargestellt sind mehrere Zeitspannen, beispielsweise die Zeitspanne $T_{70,70}$, die zwischen den folgenden beiden Zeitspannen verstreicht:

- dem Zeitpunkt $t_{i,70}$, an dem die Stromstärke I erstmals größer als 70% des Maximalwerts $I_{max}$ wird, und
- dem Zeitpunkt $t_{d,70}$, an dem sie erstmals wieder kleiner als 70% wird.

Außerdem ist die Zeitspanne $T_{70}$ eingetragen, die von Beginn der Messung (t=0) verstreicht, bis die Stromstärke I 70% des Maximalwerts $I_{max}$ erreicht. Darüber hinaus ist die Zeitspanne $T_{norm}$ eingetragen, das ist die Zeitspanne, die zwischen folgenden beiden Zeitpunkten verstreicht:

- dem Zeitpunkt $t_{i,40}$, an dem der Stromstärken-Verlauf I(t) erstmals größer als 40% des Maximalwerts $I_{max}$ wird, und
- dem Zeitpunkt $t_{d,30}$, an dem der Stromstärken-Verlauf I(t) erstmals wieder kleiner als 30% des Maximalwerts $I_{max}$ wird.

Diese Verlaufs-Parameter sind nur als Beispiele zu verstehen.

Figur 4 und Figur 5 zeigen also beispielhaft M+N unterschiedliche Verlaufs-Parameter $X_1, ..., X_M, Y_1, ..., Y_N$ des

Stromstärken-Verlaufs I(t). Nachdem das Steuergerät 6 den Stromstärken-Verlauf I(t) ermittelt hat, lässt sich für jeden Verlaufs-Parameter $X_1, ..., X_m$ jeweils ein Wert $X_1, ..., x_M$ und für jeden Verlaufs-Parameter $Y_1, ..., Y_N$ jeweils ein Wert $y_1, ..., y_N$ herleiten. Die Elektrolyt-Feuchte Ef wird in einer Ausgestaltung gemäß der Feuchte-Funktion

$$(6) \qquad Ef = [a_1{*}x_1 + ... + a_M{*}x_M] / [b_1{*}y_1 + ... + b_N{*}y_N]$$

berechnet. Die Gewichtsfaktoren $a_1, ..., a_M, b_1, ..., b_N$ werden vorab bestimmt. In einer anderen Ausgestaltung wird mit Hilfe einer Stichprobe vorab ein neuronales Netz trainiert. Die Verlaufs-Parameter $X_1, ..., X_M, Y_1, ..., Y_N$ sind Eingangs-größen dieses neuronalen Netzes.

Die Erfinder haben in internen Versuchen vorab eine Stichprobe erzeugt. Um die Stichprobe zu erzeugen, haben sie eine Kalibriervorrichtung (nicht gezeigt) verwendet, die Sensoren umfasst, welche nur für die Erzeugung der Stichprobe benötigt werden, aber nicht zum Betrieb des Analysegeräts 100. Diese Kalibriervorrichtung lässt sich mit einem elektrochemischen Sensor 12 verbinden, insbesondere mit einem elektrochemischen Sensor 12, der so aufgebaut ist wie in Figur 2 gezeigt. Die Kalibriervorrichtung empfängt ein Signal des jeweiligen Stromstärken-Sensors 38, der zu einem verbundenen elektrochemischen Sensor 12 gehört.

In einer Ausgestaltung umfasst die Kalibriervorrichtung einen Klimaschrank mit einer Klimakammer. In dieser Klimakammer lassen sich eine vorgegebene Umgebungstemperatur und eine vorgegebene Umgebungs-Feuchte einstellen. Ein elektrochemischer Sensor 12 lässt sich in diese Klimakammer verbringen. Die Elektrolyt-Feuchte Ef eines Sensors 12 in der Klimakammer stimmt nach einer Einschwingzeit mit der Umgebungs-Feuchte in der Klimakammer überein. Bevorzugt ist die Klimakammer frei von dem oder jedem zu detektierenden Zielgas.

Nacheinander werden verschiedene elektrochemische Sensoren 12 in die Klimakammer verbracht. Für jeden Sensor 12 werden nacheinander mindestens zwei verschiedene Umgebungs-Feuchten und bevorzugt auch mindestens zwei unterschiedliche Umgebungstemperaturen eingestellt. Jeder Sensor 12 liefert bei jeder Umgebungs-Feuchte und jeder Umgebungstemperatur jeweils ein Stichprobenelement. Dieses Stichprobenelement umfasst eine Elektrolyt-Feuchte Ef, nämlich die eingestellte Umgebungs-Feuchte, sowie den resultierenden Stromstärken-Verlauf I(t).

Die Stichprobe wird mit folgenden beiden Zielen ausgewertet:

- Die Elektrolyt-Feuchte Ef sollte sich mit einem Messfehler von weniger als 10% bestimmen lassen.

- Der Schätzwert für die Elektrolyt-Feuchte Ef soll relativ wenig von der Umgebungstemperatur abhängen.

Insbesondere wurden geeignete Verlaufs-Parameter $X_1, ..., X_M, Y_1, ..., Y_N$ identifiziert. Der interne Versuch lieferte folgende Ergebnisse:

Ein guter Schätzwert für die Elektrolyt-Feuchte Ef lässt sich erreichen, wenn die vier Verlaufs-Parameter $T_{70}, T_{70,35}, T_{70,70}$ und $T_{norm}$ von Figur 5 verwendet werden.

Als gute Feuchte-Funktion Ef wurde empirisch folgende Formel herausgefunden:

$$(7) \qquad Ef = [a_1{*}T_{70} + a_2{*}T_{70,70} + a_3{*}T_{70,35}] / T_{norm}.$$

Die drei Gewichtsfaktoren $a_1, a_2, a_3$ werden empirisch bestimmt, und zwar bevorzugt mit Hilfe der oben beschriebenen Stichprobe. Bevorzugt bestimmt man vorab die drei Gewichtsfaktoren $a_1, a_2, a_3$ so, dass die Abweichung zwischen der tatsächlichen Elektrolyt-Feuchte, also der Feuchte in der Klimakammer, und der Elektrolyt-Feuchte Ef, die gemäß der Feuchte-Funktion (7) ermittelt wird, minimiert wird. Ef ist ein codierter Wert aus dem Bereich von - 1 bis +1.

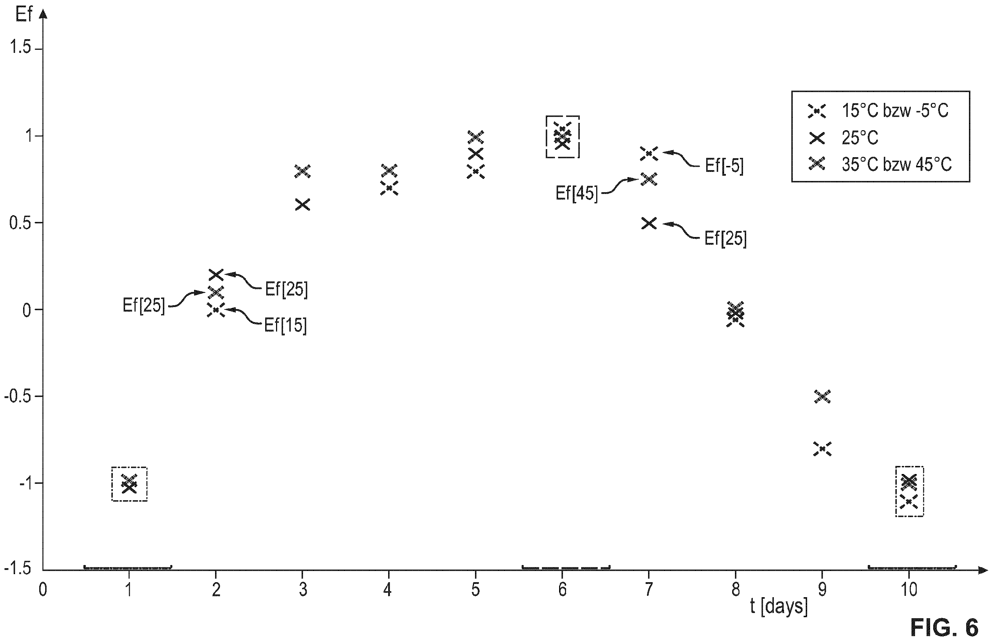

Figur 6 zeigt beispielhaft Messergebnisse. Auf der x-Achse ist die Zeit aufgetragen, auf der y-Achse die ermittelte Elektrolyt-Feuchte Ef. Die Zahlen auf der x-Achse bezeichnen Messtage. Bei den internen Versuchen verstrichen zwischen zwei aufeinanderfolgenden Messtagen mehrere Tage, an denen nicht gemessen wurde. Genau wie in Figur 3 wird die Elektrolyt-Feuchte Ef mit einem Wert aus dem Wertebereich von -1 (kleinstmögliche Elektrolyt-Feuchte, stellt sich bei der kleinstmöglichen Umgebungs-Feuchte von 0 % ein) bis +1 (größtmögliche Elektrolyt-Feuchte, stellt sich bei der größtmöglichen Umgebungs-Feuchte von 100 % ein) codiert. Um diesen codierten Wert zu ermitteln, wurde die Formel (7) verwendet. Die tatsächliche Elektrolyt-Feuchte wurde verändert, und zwar insbesondere indem die Umgebungs-Feuchte verändert wurde. Wie bereits dargelegt, führt eine kleine Umgebungs-Feuchte nach einer Einschwingzeit zu einer kleinen Elektrolyt-Feuchte, eine große Umgebungs-Feuchte zu einer großen Elektrolyt-Feuchte. Weitere Umgebungsbedingungen haben einen deutlich geringeren Einfluss auf die tatsächliche Elektrolyt-Feuchte. Um diese Einschwingzeit zu berücksichtigen, wurde die Elektrolyt-Feuchte Ef jeweils einmal an einem Messtag ermittelt, wobei zwischen zwei Messtagen mehrere Tage ohne Messung verstrichen. Falls zwischen zwei Messtagen die Umgebungs-Feuchte konstant bleibt, nimmt die tatsächliche Elektrolyt-Feuchte am späteren Messtag einen Wert an, der durch die Umgebungs-Feuchte bestimmt ist.

In den Messtagen 1 bis 6 wurden die Umgebungs-Feuchte und damit die tatsächliche Elektrolyt-Feuchte von -1 (minimaler Wert) auf +1 (maximaler Wert) vergrößert und in den Messtagen 7 bis 10 wieder auf den Minimalwert -1 verringert. An jedem Messtag wurde für drei verschiedene Umgebungstemperaturen jeweils eine Messung durchgeführt, nämlich in den Messtagen 1 bis 6 jeweils eine Messung bei +15 °C, +25 °C und +35 °C und in den Messtagen 7 bis 10 jeweils eine Messung bei -5 °C, +20 °C und +45 °C. Insgesamt wurden an jedem Messtag also jeweils drei Messungen durchgeführt.

Um die Elektrolyt-Feuchte Ef zu ermitteln, wurde die oben angegebene Formel (7) mit vorab empirisch ermittelten Gewichtsfaktoren $a_1$, $a_2$, $a_3$ angewendet. Mit Ef[Temp] wird die ermittelte Codierung der Elektrolyt-Feuchte Ef bei der Umgebungstemperatur Temp bezeichnet. Ef[Temp] ist also eine Zahl aus dem Intervall [-1,+1].

Folgende Ergebnisse wurden erzielt:

- Jede ermittelte Elektrolyt-Feuchte Ef wich um maximal 15 % nach oben oder nach unten von der tatsächlichen Elektrolyt-Feuchte bei der jeweiligen Umgebungstemperatur Temp ab. Genauer gesagt: Die erfindungsgemäß ermittelte Codierung für die Elektrolyt-Feuchte (ein Wert zwischen -1 und +1) wich um maximal 15 % von der Elektrolyt-Feuchte ab, die sich bei der eingestellten Umgebungs-Feuchte einstellt. Dies ist für viele Anwendungen eine ausreichende Genauigkeit.
- Wie in Figur 6 zu sehen ist, hat die Umgebungstemperatur Temp einen relativ geringen Einfluss auf die ermittelte Elektrolyt-Feuchte Ef. Der Einfluss der Umgebungstemperatur auf das Ermittlungs-Ergebnis konnte also rechnerisch weitgehend eliminiert werden.

Wie bereits dargelegt, ist in einer Ausgestaltung der elektrochemische Sensor 12 von Figur 2 ein Bestandteil eines Analysegeräts 100. Das Analysegerät 100 leitet eine Messkammer-Probe Pr in die Messkammer 3, und der elektrochemische Sensor 12 analysiert diese Messkammer-Probe Pr. Das Steuergerät 6 ermittelt den Stromstärken-Verlauf I(t), der in Figur 4 und Figur 5 beispielhaft gezeigt wird. Das Steuergerät 6 leitet die gesamte elektrische Ladung Q = $Q_{meas}$ her und entscheidet abhängig von der Ladung $Q_{meas}$, ob die Messkammer-Probe Pr Atemalkohol oberhalb einer vorgegebenen unteren Konzentrations-Schranke aufweist oder nicht. Für diese Entscheidung wird die Kenntnis der Elektrolyt-Feuchte Ef nicht benötigt.

Die Detektion des Ereignisses, dass die Messkammer-Probe Pr Atemalkohol oberhalb der Konzentrations-Schranke aufweist, löst in einer Ausgestaltung einerseits den Schritt aus, dass eine entsprechende Meldung in mindestens einer von einem Menschen wahrnehmbaren Form ausgegeben wird.

Die gerade beschriebene Detektion löst außerdem die folgenden Schritte aus: Das Steuergerät 6 ermittelt die aktuelle Feuchte Ef des Elektrolyten 28. Beispielsweise ermittelt das Steuergerät 6 den jeweiligen Wert von mehreren Verlaufs-Parametern des Stromstärken-Verlaufs I(t) und wendet eine Feuchte-Funktion an, beispielsweise die Feuchte-Funktion (6). Die vorab bestimmten Gewichtsfaktoren $a_1$, $a_2$, $a_3$ sind Bestandteil eines Programms, welches das Steuergerät 6 anwendet, oder sind im Datenspeicher 7 des Analysegeräts 100 abgespeichert.

Bei einer konstanten Konzentration con des Zielgases, hier des Atemalkohols, ist die gemessene Ladung $Q_{meas}$ umso größer, je größer die Elektrolyt-Feuchte Ef ist, vgl. die Rechenvorschrift (4). Diese Abhängigkeit lässt sich durch einen funktionalen Zusammenhang beschreiben. Beispielsweise gilt mit ausreichender Genauigkeit der lineare Zusammenhang (5). Das Steuergerät 6 verwendet die ermittelte Elektrolyt-Feuchte Ef, um den Einfluss der Elektrolyt-Feuchte Ef auf die Messung zu kompensieren. Bevorzugt leitet das Steuergerät 6 eine korrigierte Ladung $Q_{korr}$ her, und zwar gemäß der Rechenvorschrift

$$(8) \qquad Q_{korr} = G^{-1}(Ef)*Q_{meas}$$

$G^{-1}$ ist die Umkehrfunktion zur Funktion G = G(Ef) in der Rechenvorschrift (4).

Die gesuchte Zielgas-Konzentration con wird ermittelt, indem der Zusammenhang F in der Rechenvorschrift (1) auf die korrigierte Spannung $Q_{korr}$ angewendet wird. Beispielsweise gilt

$$(9) \qquad con = \alpha*Q_{korr}.$$

Außerdem detektiert das Steuergerät 6 das unerwünschte Ereignis, dass die Elektrolyt-Feuchte Ef für eine ausreichend lange Zeitspanne außerhalb eines vorgegebenen Soll-Wertebereichs liegt. Dieses Ereignis kann ein Indiz dafür sein, dass Elektrolyt 28 verdampft ist und der Sensor 12 trotz der gerade beschriebenen rechnerischen Kompensation nicht mehr in der Lage ist, zuverlässige Messergebnisse zu generieren. Bevorzugt bewirkt das Steuergerät 6, dass eine Nachricht mit diesem Ereignis generiert und in mindestens einer von einem Menschen wahrnehmbaren Form ausgegeben wird.

**Bezugszeichenliste**

| 1 | Mundstück, lässt sich lösbar auf die Röhre 2 aufsetzen |
|---|---|
| 2 | Röhre, lässt sich lösbar mit dem Gehäuse 4 verbinden, trägt das Mundstück 1 |
| 3 | Messkammer, nimmt den elektrochemischen Sensor 12 auf |
| 4 | Gehäuse, nimmt die Messkammer 3, den Sensor 12, das Steuergerät 6 und den Datenspeicher 7 auf, trägt die Röhre 2 |
| 6 | Steuergerät, empfängt und verarbeitet ein Signal des Stromstärken-Sensors 38 und optional ein Signal des Temperatursensors 52, ermittelt den Stromstärken-Verlauf I(t), die elektrische Ladung $Q_{meas}$ und die Elektrolyt-Feuchte Ef |
| 7 | Datenspeicher, auf den das Steuergerät 6 wenigstens zeitweise Lesezugriff hat |
| 12 | elektrochemischer Sensor 12, umfasst die Elektroden 20 und 21, die Kontaktierungsdrähte 33 und 34 und den Elektrolyten 28 |
| 20 | Messelektrode, vom Kontaktierungsdraht 34 elektrisch kontaktiert |
| 21 | Gegenelektrode, vom Kontaktierungsdraht 33 elektrisch kontaktiert |
| 22 | Verbindungsdraht zwischen den Kontaktierungsdrähten 33 und 34 |
| 28 | Elektrolyt zwischen den beiden Elektroden 20 und 21, weist die Feuchte Ef auf |
| 29 | elektrischer Messwiderstand im Verbindungsdraht 22 |
| 33 | Kontaktierungsdraht für die Gegenelektrode 21 |
| 34 | Kontaktierungsdraht für die Messelektrode 20 |
| 38 | Stromstärken-Sensor, misst die Stärke des Stroms, der durch den Verbindungsdraht 22 fließt |
| 40 | Wandung für die Messkammer 3 |
| 50 | Sensor-Anordnung, umfasst den elektrochemischen Sensor 12, die Messkammer 3 und die Wandung 40 |
| 52 | optionaler Temperatursensor, misst die Temperatur in der Umgebung der Sensor-Anordnung 50 |
| 100 | Analysegerät, umfasst das Gehäuse 4, die Sensor-Anordnung 50 mit dem elektrochemischen Sensor 12 und der Messkammer 3, das Steuergerät 6, den Datenspeicher 7, die Röhre 2 und das Mundstück 1 sowie eine nicht gezeigte Spannungsversorgungseinheit |
| A | Atemprobe, wird in das Mundstück 1 eingegeben, fließt durch die Röhre 2 |
| Ef | aktuelle Feuchte des Elektrolyten 28, wird vom Steuergerät 6 ermittelt |
| Ef[Temp] | ermittelte Elektrolyt-Feuchte bei der Umgebungstemperatur Temp |
| G | funktionaler Zusammenhang, liefert die elektrische Ladung als Funktion der elektrischen Ladung bei einer Referenz-Elektrolyt-Feuchte und der tatsächlichen Elektrolyt-Feuchte |
| I(t) | zeitlicher Verlauf der Stärke I des Stroms, der durch die Verbindungsdraht 22 fließt, wird abhängig von einem Signal des Stromstärken-Sensors 38 ermittelt |
| I([Ef,Temp](t) | zeitlicher Verlauf der Stromstärke I bei einer Elektrolyt-Feuchte Ef und eine Umgebungstemperatur von Temp |
| $I_{max}$ | Maximalwert des Stromstärken-Verlaufs I(t), wird zum Zeitpunkt $t_{max}$ angenommen |
| I'(twp) | zeitliche Veränderung der Stromstärke I zum Zeitpunkt $t_{Wp}$ |
| MA | Mittelachse der Wandung 40 |
| Ö.a | austrittsseitige Öffnung aus der Messkammer 3 |
| Ö.e | eintrittsseitige Öffnung in die Messkammer 3 |

(fortgesetzt)

| | |
|---|---|
| Pr | Messkammer-Probe, wird von der Atemprobe A abgezweigt, die durch die Röhre 2 fließt, gelangt in die Messkammer 3, kann Atemalkohol als das zu detektierenden Zielgas enthalten |
| $Q_k$ | elektrische Ladung, die zwischen den Zeitpunkten $t_1$ und $t_2$ fließt |
| $Q_{korr}$ | unter Verwendung der ermittelten Elektrolyt-Feuchte Ef korrigierter Wert für die elektrische Ladung $Q_{meas}$ |
| $Q_{meas}$ | aus dem gemessenen Stromstärken-Verlauf I(t) hergeleitete elektrische Ladung |
| $Q_{ref}$ | Referenzwert für die elektrische Ladung Q, wird unter vorgegebenen Referenzbedingungen erzielt, insbesondere bei einem Referenzwert für die Elektrolyt-Feuchte Ef |
| Sk | Sekante zwischen den beiden Punkten $[t_a, I(t_a)]$ und $t_b, I(t_b)]$ |
| $t_{d,y}$ | spätester Zeitpunkt, an denen die Stromstärke I(t) y% der maximalen Stromstärke $I_{max}$ beträgt |
| Temp | Umgebungstemperatur |
| $t_{i,x}$ | frühster Zeitpunkt, an denen der Stromstärken-Verlauf I(t) x% der maximalen Stromstärke $I_{max}$ beträgt |
| $t_{max}$ | Zeitpunkt, an dem der Stromstärken-Verlauf I(t) den Maximalwert $I_{max}$ annimmt |
| $t_{Wp}$ | Zeitpunkt, an dem der Stromstärken-Verlauf I(t) den Wendepunkt Wp und die Steigung $I'(t_{Wp})$ aufweist |
| $T_x$ | Zeitspanne zwischen dem Beginn der Messung (t=0) und dem Zeitpunkt $t_{i,x}$ |
| $T_{x,y}$ | Zeitspanne zwischen den beiden Zeitpunkten $t_{i,x}$ und $t_{d,y}$ |
| $T_{norm}$ | Zeitspanne zwischen den beiden Zeitpunkten $t_{i,40}$ und $t_{d,30}$ |
| Wp | Wendepunkt des Stromstärken-Verlaufs I(t), wird zum Zeitpunkt $t_{Wp}$ angenommen |

**Patentansprüche**

1.  Überprüfungs-Einrichtung (6, 7) zum Überprüfen eines elektrochemischen Sensors (12),

    wobei der elektrochemische Sensor (12)

        - eine Messkammer (3) zum Aufnehmen einer Gasprobe (Gp), zwei Elektroden (20, 21) und einen ionisch leitenden Elektrolyten (28) zwischen den beiden Elektroden (20, 21) umfasst und
        - nach Art einer Brennstoffzelle so ausgestaltet ist, dass ein Zielgas in der Messkammer (3) eine elektrochemische Reaktion hervorruft, die das Fließen eines elektrischen Stroms (I) bewirkt,

    wobei eine messbare Detektionsgröße des fließenden elektrischen Stroms (I), insbesondere die elektrische Ladung (Q), mit einer Konzentration des Zielgases in einer Gasprobe (Gp) in der Messkammer (3) korreliert,
    wobei der Elektrolyt (28) eine zeitlich veränderliche Feuchte (Ef) aufweist, wobei mehrere Verlaufs-Parameter $[T_{70}, T_{70,35}, T_{70,70}, T_{norm}]$ und eine rechnerauswertbare Feuchte-Funktion vorgegeben sind,
    wobei jeder vorgegebene Verlaufs-Parameter $[T_{70}, T_{70,35}, T_{70,70}, T_{norm}]$ ein Parameter eines zeitlichen Verlaufs [I(t)] einer Stromstärke ist,
    wobei die vorgegebene rechnerauswertbare Feuchte-Funktion die Feuchte (Ef) des Elektrolyten (28) als Funktion der vorgegebenen Verlaufs-Parameter $[T_{70}, T_{70,35}, T_{70,70}, T_{norm}]$ beschreibt,
    wobei die Überprüfungs-Einrichtung (6, 7) ein signalverarbeitendes Steuergerät (6) umfasst,
    wobei das Steuergerät (6) dazu ausgestaltet ist,

        - einen tatsächlichen Stromstärken-Verlauf [I(t)] zu ermitteln, das ist der zeitliche Verlauf der Stärke (I) des Stroms, dessen Fließen durch die elektrochemische Reaktion hervorgerufen wird, und
        - die aktuelle Feuchte (Ef) des Elektrolyten (28) unter Verwendung des tatsächlichen Stromstärken-Verlaufs [I(t)] zu ermitteln,

    wobei das Steuergerät (6) dazu ausgestaltet ist, für die Ermittlung der aktuellen Feuchte (Ef) des Elektrolyten (28)

- für jeden vorgegebenen Verlaufs-Parameter [$T_{70}$, $T_{70,35}$, $T_{70,70}$, $T_{norm}$] zu ermitteln, welchen Wert dieser Verlaufs-Parameter [$T_{70}$, $T_{70,35}$, $T_{70,70}$, $T_{norm}$] für den ermittelten tatsächlichen Stromstärken-Verlauf [$I(t)$] annimmt, und
- die vorgegebene Feuchte-Funktion auf die ermittelten Verlaufs-Parameter-Werte anzuwenden.

2.  Überprüfungs-Einrichtung (6, 7) nach Anspruch 1,

**dadurch gekennzeichnet, dass**
mindestens ein Verlaufs-Parameter des Stromstärken-Verlaufs [$I(t)$]
die Zeitspanne [$T_{70}$, $T_{70,35}$, $T_{70,70}$, $T_{norm}$], die zwischen

- dem Beginn der Messung und
- dem frühesten oder spätesten Zeitpunkt ($t_{i,70}$), an dem der Stromstärken-Verlauf [$I(t)$] gleich $x_1$% der maximalen Stromstärke ($I_{max}$) wird, verstreicht,

wobei $x_1$ und $x_2$ zwei Prozentwerte sind, die miteinander übereinstimmen oder voneinander verschieden sind, oder
die Zeitspanne, die zwischen

- dem frühesten oder spätesten Zeitpunkt ($t_{i,40}$, $t_{i,70}$), an dem der Stromstärken-Verlauf [$I(t)$] gleich $x_1$% der maximalen Stromstärke ($I_{max}$) wird, und
- dem frühesten oder spätesten Zeitpunkt ($t_{d,30}$, $t_{d,70}$), an dem der Stromstärken-Verlauf [$I(t)$] gleich $x_2$% der maximalen Stromstärke ($I_{max}$) wird,

verstreicht, oder
die elektrische Ladung ($Q_k$), die zwischen

- dem Beginn der Messung und
- dem frühesten oder spätesten Zeitpunkt ($t_{i,70}$), an dem der Stromstärken-Verlauf [$I(t)$] gleich $x_1$% der maximalen Stromstärke ($I_{max}$) wird,

fließt, oder
die elektrische Ladung ($Q_k$), die zwischen

- dem frühesten oder spätesten Zeitpunkt ($t_{i,40}$, $t_{i,70}$), an dem der Stromstärken-Verlauf [$I(t)$] gleich $x_1$% der maximalen Stromstärke ($I_{max}$) wird, und
- dem frühesten oder spätesten Zeitpunkt ($t_{d,30}$, $t_{d,70}$), an dem der Stromstärken-Verlauf [$I(t)$] gleich $x_2$% der maximalen Stromstärke ($I_{max}$) wird,

fließt, oder
die Steigung einer Sekante (Sk) zwischen

- dem Beginn der Messung und dem frühesten oder spätesten Zeitpunkt ($t_{i,70}$), an dem der Stromstärken-Verlauf [$I(t)$] gleich $x_1$% der maximalen Stromstärke ($I_{max}$) wird, oder
- dem frühesten oder spätesten Zeitpunkt ($t_{i,40}$, $t_{i,70}$), an dem der Stromstärken-Verlauf [$I(t)$] gleich $x_1$% der maximalen Stromstärke ($I_{max}$) wird, und dem frühesten oder spätesten Zeitpunkt ($t_{d,30}$, $t_{d,70}$), an dem der Stromstärken-Verlauf [$I(t)$] gleich $x_2$% der maximalen Stromstärke ($I_{max}$) wird.

3.  Überprüfungs-Einrichtung (6, 7) nach einem der vorhergehenden Ansprüche, **dadurch gekennzeichnet, dass**

ein Verlaufs-Parameter des Stromstärken-Verlaufs [$I(t)$] die Zeitspanne ($T_{norm}$) zwischen den folgenden beiden Zeitpunkten ist:

- dem frühesten Zeitpunkt, an dem der Stromstärken-Verlauf [$I(t)$] gleich $x_1$% der maximalen Stromstärke ($I_{max}$) wird, und
- dem spätesten Zeitpunkt, an dem der Stromstärken-Verlauf [$I(t)$] gleich $x_2$% der maximalen Stromstärke ($I_{max}$) wird,
wobei $x_1$ und $x_2$ zwei Prozentwerte sind, die miteinander übereinstimmen oder voneinander verschieden

sind,

wobei bevorzugt $x_1$ größer als $x_2$ ist,
wobei die Feuchte-Funktion ein Quotient aus einer Zähler-Feuchte-Funktion und einer Nenner-Feuchte-Funktion ist,
wobei in der Nenner-Feuchte-Funktion die Zeitspanne ($T_{norm}$) zwischen dem frühesten und dem spätesten Zeitpunkt auftritt und
wobei in der Zähler-Feuchte-Funktion mindestens ein anderer Verlaufs-Parameter [$T_{70}$, $T_{70,35}$, $T_{70,70}$] des Stromstärken-Verlaufs [I(t)] auftritt.

4.  Überprüfungs-Einrichtung (6, 7) nach Anspruch 3,

    **dadurch gekennzeichnet, dass**
    die Nenner-Feuchte-Funktion gleich der Zeitspanne ($T_{norm}$) zwischen dem frühesten und dem spätesten Zeitpunkt ist und
    die Zähler-Feuchte-Funktion ein gewichtetes Mittel von mindestens zwei anderen Verlaufs-Parametern [$T_{70}$, $T_{70,35}$, $T_{70,70}$] des Stromstärken-Verlaufs [I(t)] ist.

5.  Überprüfungs-Einrichtung (6, 7) nach einem der vorhergehenden Ansprüche, **dadurch gekennzeichnet, dass**

    das Steuergerät (6) dazu ausgestaltet ist, ein Signal von einem Temperatursensor (52) zu empfangen,
    wobei das Signal eine vom Temperatursensor (52) gemessene Temperatur in einer Umgebung des elektrochemischen Sensors (12) beschreibt und wobei die vorgegebene Feuchte-Funktion die Feuchte (Ef) des Elektrolyten (28) als Funktion der vorgegebenen Verlaufs-Parameter [$T_{70}$, $T_{70,35}$, $T_{70,70}$, $T_{norm}$] und der Umgebungstemperatur beschreibt.

6.  Analysegerät (100) zum Messen einer Konzentration eines Zielgases in einer Gasprobe (Pr),

    wobei das Analysegerät (100)

    - eine Messkammer (3),
    - zwei Elektroden (20, 21) und
    - einen ionisch leitenden Elektrolyten (28) zwischen den beiden Elektroden (20, 21)

    umfasst,
    wobei der Elektrolyt (28) eine zeitlich veränderliche Feuchte (Ef) aufweist,
    wobei die Messkammer (3) dazu ausgestaltet ist, die Gasprobe (Pr) aufzunehmen,
    wobei das Analysegerät (100) so ausgestaltet ist, dass

    - das Zielgas als Bestandteil der Gasprobe (Pr) in der Messkammer (3) eine elektrochemische Reaktion nach Art einer Brennstoffzelle hervorruft und
    - die bewirkte elektrochemische Reaktion das Fließen eines elektrischen Stroms (I) zwischen den beiden Elektroden (20, 21) bewirkt,

    wobei das Analysegerät (100) dazu ausgestaltet ist,

    - eine Detektionsgröße des fließenden elektrischen Stroms (I), insbesondere die elektrische Ladung (Q), zu messen, wobei die Detektionsgröße mit der Konzentration des Zielgases in einer Gasprobe (Gp) in der Messkammer (3) korreliert, und
    - die Zielgas-Konzentration abhängig von der gemessenen Detektionsgröße (Q) des fließenden elektrischen Stroms zu messen, wobei mehrere Verlaufs-Parameter [$T_{70}$, $T_{70,35}$, $T_{70,70}$, $T_{norm}$] und eine rechnerauswertbare Feuchte-Funktion vorgegeben sind,

    wobei jeder vorgegebene Verlaufs-Parameter [$T_{70}$, $T_{70,35}$, $T_{70,70}$, $T_{norm}$] ein Parameter eines zeitlichen Verlaufs [I(t)] einer Stromstärke ist,
    wobei die vorgegebene rechnerauswertbare Feuchte-Funktion die Feuchte (Ef) des Elektrolyten (28) als Funktion der vorgegebenen Verlaufs-Parameter [$T_{70}$, $T_{70,35}$, $T_{70,70}$, $T_{norm}$] beschreibt,
    wobei das Analysegerät (100) dazu ausgestaltet ist,

- einen tatsächlichen Stromstärken-Verlauf [I(t)] zu ermitteln, das ist der zeitliche Verlauf der Stärke (I) des Stroms, dessen Fließen durch die elektrochemische Reaktion hervorgerufen wird, und
- die aktuelle Feuchte des Elektrolyten (28) unter Verwendung des tatsächlichen Stromstärken-Verlaufs [I(t)] zu ermitteln,

wobei das Analysegerät (100) weiterhin dazu ausgestaltet ist, für die Ermittlung der aktuellen Feuchte (Ef) des Elektrolyten (28)

- für jeden vorgegebenen Verlaufs-Parameter [$T_{70}$, $T_{70,35}$, $T_{70,70}$, $T_{norm}$] zu ermitteln, welchen Wert dieser Verlaufs-Parameter [$T_{70}$, $T_{70,35}$, $T_{70,70}$, $T_{norm}$] für den ermittelten tatsächlichen Stromstärken-Verlauf [I(t)] annimmt, und
- die vorgegebene Feuchte-Funktion auf die ermittelten Verlaufs-Parameter-Werte anzuwenden.

7. Analysegerät (100) nach Anspruch 6,
**dadurch gekennzeichnet, dass**

in rechnerauswertbarer Form eine Konzentrations-Funktion vorgegeben ist,
wobei die Konzentrations-Funktion die Zielgas-Konzentration als Funktion der Detektionsgröße und der Elektrolyt-Feuchte (Ef) beschreibt, und
das Analysegerät (100) dazu ausgestaltet ist,
bei der Messung der Zielgas-Konzentration die vorgegebene Konzentrations-Funktion auf die gemessene Detektionsgröße (Q) und die ermittelte Elektrolyt-Feuchte (Ef) anzuwenden.

8. Analysegerät (100) nach Anspruch 6 oder Anspruch 7,
**dadurch gekennzeichnet, dass**

das Analysegerät (100) dazu ausgestaltet ist,
abhängig von der gemessenen Detektionsgröße (Q) des fließenden elektrischen Stroms (I) zu entscheiden, ob die Gasprobe (Pr) das Zielgas mit einer Konzentration innerhalb oder außerhalb eines vorgegebenen unteren Konzentrations-Wertebereichs aufweist oder nicht, und
mindestens dann, wenn die Zielgas-Konzentration außerhalb des Konzentrations-Wertebereichs liegt, die Elektrolyt-Feuchte (Ef) zu ermitteln.

9. Überprüfungs-Verfahren zum Überprüfen eines elektrochemischen Sensors (12)

wobei der elektrochemische Sensor (12)

- eine Messkammer (3) zum Aufnehmen einer Gasprobe (Gp), zwei Elektroden (20, 21) und einen ionisch leitenden Elektrolyten (28) zwischen den beiden Elektroden (20, 21) umfasst und
- nach Art einer Brennstoffzelle so ausgestaltet ist, dass ein Zielgas in der Messkammer (3) eine elektrochemische Reaktion hervorruft, die das Fließen eines elektrischen Stroms (I) bewirkt,

wobei der Elektrolyt (28) eine zeitlich veränderliche Feuchte (Ef) aufweist,
wobei mehrere Verlaufs-Parameter [$T_{70}$, $T_{70,35}$, $T_{70,70}$, $T_{norm}$] und eine rechnerauswertbare Feuchte-Funktion vorgegeben werden,
wobei jeder vorgegebene Verlaufs-Parameter [$T_{70}$, $T_{70,35}$, $T_{70,70}$, $T_{norm}$] ein Parameter eines zeitlichen Verlaufs [I(t)] einer Stromstärke ist,
wobei die vorgegebene rechnerauswertbare Feuchte-Funktion die Feuchte (Ef) des Elektrolyten (28) als Funktion der vorgegebenen Verlaufs-Parameter [$T_{70}$, $T_{70,35}$, $T_{70,70}$, $T_{norm}$] beschreibt,
wobei das Überprüfungs-Verfahren unter Verwendung eines signalverarbeitenden Steuergeräts (6) durchgeführt wird und
die Schritte umfasst, dass das Steuergerät (6)

- einen tatsächlichen Stromstärken-Verlauf [I(t)] ermittelt, das ist der zeitliche Verlauf der Stärke (I) des Stroms, dessen Fließen durch die elektrochemische Reaktion hervorgerufen wird, und
- die aktuelle Feuchte (Ef) des Elektrolyten (28) unter Verwendung des ermittelten tatsächlichen Stromstärken-Verlaufs [I(t)] ermittelt,

wobei das Überprüfungs-Verfahren die weiteren Schritte umfasst, dass das Steuergerät (6) für die Ermittlung der aktuellen Feuchte (Ef) des Elektrolyten (28)

- für jeden vorgegebenen Verlaufs-Parameter [$T_{70}$, $T_{70,35}$, $T_{70,70}$, $T_{norm}$] ermittelt, welchen Wert dieser Verlaufs-Parameter [$T_{70}$, $T_{70,35}$, $T_{70,70}$, $T_{norm}$] für den ermittelten tatsächlichen Stromstärken-Verlauf [I(t)] annimmt, und
- die vorgegebene Feuchte-Funktion auf die ermittelten Verlaufs-Parameter-Werte anwendet.

10. Überprüfungs-Verfahren nach Anspruch 9,
**dadurch gekennzeichnet, dass**

mindestens ein Verlaufs-Parameter des Stromstärken-Verlaufs [I(t)]
die Zeitspanne [$T_{70}$, $T_{70,35}$, $T_{70,70}$, $T_{norm}$] zwischen den folgenden beiden Zeitpunkten ist:

- dem Beginn der Messung und dem frühesten Zeitpunkt ($t_{i,70}$), an dem der Stromstärken-Verlauf [I(t)] gleich $x_1$% der maximalen Stromstärke ($I_{max}$) wird, oder
- dem frühesten oder spätesten Zeitpunkt ($t_{i,40}$, $t_{i,70}$), an dem der Stromstärken-Verlauf [I(t)] gleich $x_1$% der maximalen Stromstärke ($I_{max}$) wird, und dem frühesten oder spätesten Zeitpunkt ($t_{d,30}$, $t_{d,70}$), an dem der Stromstärken-Verlauf [I(t)] gleich $x_2$% der maximalen Stromstärke ($I_{max}$) wird,
wobei $x_1$ und $x_2$ zwei Prozentwerte sind, die miteinander übereinstimmen oder voneinander verschieden sind, oder

die elektrische Ladung ($Q_k$), die zwischen

- dem frühesten oder spätesten Zeitpunkt ($t_{i,40}$, $t_{i,70}$), an dem der Stromstärken-Verlauf [I(t)] gleich $x_1$% der maximalen Stromstärke ($I_{max}$) wird, und
- dem frühesten oder spätesten Zeitpunkt ($t_{d,30}$, $t_{d,70}$), an dem der Stromstärken-Verlauf [I(t)] gleich $x_2$% der maximalen Stromstärke ($I_{max}$) wird,

fließt, oder
die Steigung einer Sekante (Sk) zwischen

- dem frühesten oder spätesten Zeitpunkt ($t_{i,40}$, $t_{i,70}$), an dem der Stromstärken-Verlauf [I(t)] gleich $x_1$% der maximalen Stromstärke ($I_{max}$) wird, und
- dem frühesten oder spätesten Zeitpunkt ($t_{d,30}$, $t_{d,70}$), an dem der Stromstärken-Verlauf [I(t)] gleich $x_2$% der maximalen Stromstärke ($I_{max}$) wird.

11. Mess-Verfahren zum Messen einer Konzentration eines Zielgases in einer Gasprobe (Pr)

unter Verwendung eines elektrochemischen Sensors (12),
wobei der elektrochemische Sensor (12)

- eine Messkammer (3),
- zwei Elektroden (20, 21) und
- einen ionisch leitenden Elektrolyten (28) zwischen den beiden Elektroden (20, 21)

umfasst,
wobei der Elektrolyt (28) eine zeitlich veränderliche Feuchte (Ef) aufweist,
wobei mehrere Verlaufs-Parameter [$T_{70}$, $T_{70,35}$, $T_{70,70}$, $T_{norm}$] und eine rechnerauswertbare Feuchte-Funktion vorgegeben werden,
wobei jeder vorgegebene Verlaufs-Parameter [$T_{70}$, $T_{70,35}$, $T_{70,70}$, $T_{norm}$] ein Parameter eines zeitlichen Verlaufs [I(t)] einer Stromstärke ist,
wobei die vorgegebene rechnerauswertbare Feuchte-Funktion die Feuchte (Ef) des Elektrolyten (28) als Funktion der vorgegebenen Verlaufs-Parameter [$T_{70}$, $T_{70,35}$, $T_{70,70}$, $T_{norm}$] beschreibt,
wobei das Mess-Verfahren die Schritte umfasst, dass

- ermöglicht oder bewirkt wird, dass die Gasprobe (Pr) in die Messkammer (3) fließt,
- das Zielgas als Bestandteil der Gasprobe (Pr) in der Messkammer (3) eine elektrochemische Reaktion nach Art einer Brennstoffzelle hervorruft,
- die bewirkte elektrochemische Reaktion das Fließen eines elektrischen Stroms (I) zwischen den beiden Elektroden (20, 21) bewirkt,
- eine Detektionsgröße des fließenden elektrischen Stroms (I), insbesondere die elektrische Ladung (Q), gemessen wird und
- die Zielgas-Konzentration abhängig von der gemessenen Detektionsgröße (Q) des fließenden elektrischen Stroms gemessen wird,

wobei das Mess-Verfahren die weiteren Schritte umfasst, dass

- ein tatsächlicher Stromstärken-Verlauf [I(t)] ermittelt wird, das ist der zeitliche Verlauf der Stärke (I) des Stroms, dessen Fließen durch die elektrochemische Reaktion hervorgerufen wird, und
- die aktuelle Feuchte (Ef) des Elektrolyten (28) unter Verwendung des ermittelten tatsächlichen Stromstärken-Verlaufs [I(t)] ermittelt wird, wobei der Schritt, die aktuelle Elektrolyt-Feuchte (Ef) zu ermitteln, die Schritte umfasst, dass
- für jeden vorgegebenen Verlaufs-Parameter [$T_{70}$, $T_{70,35}$, $T_{70,70}$, $T_{norm}$] ermittelt wird, welchen Wert dieser Verlaufs-Parameter [$T_{70}$, $T_{70,35}$, $T_{70,70}$, $T_{norm}$] für den ermittelte Stromstärken-Verlauf [I(t)] annimmt, und
- die vorgegebene Feuchte-Funktion auf die ermittelten Verlaufs-Parameter-Werte angewendet wird.

**12.** Mess-Verfahren nach Anspruch 11,
**dadurch gekennzeichnet, dass**

in rechnerauswertbarer Form eine Konzentrations-Funktion vorgegeben wird,
wobei die Konzentrations-Funktion die Zielgas-Konzentration als Funktion der Detektionsgröße (Q) und der Elektrolyt-Feuchte (Ef) beschreibt, und
der Schritt, die Zielgas-Konzentration zu messen, den Schritt umfasst, dass
die vorgegebene Konzentrations-Funktion auf die gemessene Detektionsgröße (Q) und die ermittelte Elektrolyt-Feuchte (Ef) angewendet wird.

**13.** Mess-Verfahren nach Anspruch 11 oder Anspruch 12,
**dadurch gekennzeichnet, dass**

abhängig von der gemessenen Detektionsgröße (Q) des fließenden elektrischen Stroms entschieden wird, ob die Gasprobe (Pr) das Zielgas mit einer Konzentration außerhalb eines vorgegebenen unteren Konzentrations-Wertebereichs aufweist oder nicht, und
die Detektion, dass die Gasprobe (Pr) das Zielgas mit einer Konzentration außerhalb des Konzentrations-Wertebereichs aufweist, den Schritt auslöst, dass die aktuelle Elektrolyt-Feuchte (Ef) ermittelt wird.

**Geänderte Patentansprüche gemäss Regel 137(2) EPÜ.**

**1.** Analysegerät (100) zum Messen einer Konzentration eines Zielgases in einer Gasprobe (Pr),

wobei das Analysegerät (100)

- eine Messkammer (3),
- zwei Elektroden (20, 21)
- einen ionisch leitenden Elektrolyten (28) zwischen den beiden Elektroden (20, 21),
- einen Stromstärken-Sensor (38) und
- eine Überprüfungs-Einrichtung (6, 7)

umfasst,
wobei der Elektrolyt (28) eine zeitlich veränderliche Feuchte (Ef) aufweist,
wobei die Messkammer (3) dazu ausgestaltet ist, die Gasprobe (Pr) aufzunehmen,
wobei das Analysegerät (100) so ausgestaltet ist, dass

- das Zielgas als Bestandteil der Gasprobe (Pr) in der Messkammer (3) eine elektrochemische Reaktion nach Art einer Brennstoffzelle hervorruft und
- die bewirkte elektrochemische Reaktion das Fließen eines elektrischen Stroms (I) zwischen den beiden Elektroden (20, 21) bewirkt,

wobei der Stromstärken-Sensor (38) dazu ausgestaltet ist, wiederholt die aktuelle Stärke des fließenden elektrischen Stroms (I) zu messen,
wobei das Analysegerät (100) dazu ausgestaltet ist,

- eine Detektionsgröße des fließenden elektrischen Stroms (I), insbesondere die elektrische Ladung (Q), zu messen, wobei die Detektionsgröße mit der Konzentration des Zielgases in einer Gasprobe (Gp) in der Messkammer (3) korreliert, und
- die Zielgas-Konzentration abhängig von der gemessenen Detektionsgröße (Q) des fließenden elektrischen Stroms zu messen,

wobei die Überprüfungs-Einrichtung (6, 7) ein signalverarbeitendes Steuergerät (6) umfasst,
wobei mehrere Verlaufs-Parameter [$T_{70}$, $T_{70,35}$, $T_{70,70}$, $T_{norm}$] und eine rechnerauswertbare Feuchte-Funktion vorgegeben sind,
wobei jeder vorgegebene Verlaufs-Parameter [$T_{70}$, $T_{70,35}$, $T_{70,70}$, $T_{norm}$] ein Parameter eines zeitlichen Verlaufs [$I(t)$] einer Stromstärke ist,
wobei die vorgegebene rechnerauswertbare Feuchte-Funktion die Feuchte (Ef) des Elektrolyten (28) als Funktion der vorgegebenen Verlaufs-Parameter [$T_{70}$, $T_{70,35}$, $T_{70,70}$, $T_{norm}$] beschreibt,
wobei das Steuergerät (6) dazu ausgestaltet ist,

- vom Stromstärken-Sensor (38) ein Signal zu empfangen, welches die gemessene Stromstärke (I) beschreibt,
- einen tatsächlichen Stromstärken-Verlauf [$I(t)$] zu ermitteln, das ist der zeitliche Verlauf der Stärke (I) des Stroms, dessen Fließen durch die elektrochemische Reaktion hervorgerufen wird, und
- die aktuelle Feuchte des Elektrolyten (28) unter Verwendung des tatsächlichen Stromstärken-Verlaufs [$I(t)$] zu ermitteln,

wobei das Steuergerät (6) weiterhin dazu ausgestaltet ist, für die Ermittlung der aktuellen Feuchte (Ef) des Elektrolyten (28)

- für jeden vorgegebenen Verlaufs-Parameter [$T_{70}$, $T_{70,35}$, $T_{70,70}$, $T_{norm}$] zu ermitteln, welchen Wert dieser Verlaufs-Parameter [$T_{70}$, $T_{70,35}$, $T_{70,70}$, $T_{norm}$] für den ermittelten tatsächlichen Stromstärken-Verlauf [$I(t)$] annimmt, und
- die vorgegebene Feuchte-Funktion auf die ermittelten Verlaufs-Parameter-Werte anzuwenden.

2. Analysegerät (100) nach Anspruch 1,
**dadurch gekennzeichnet, dass**

in rechnerauswertbarer Form eine Konzentrations-Funktion vorgegeben ist,
wobei die Konzentrations-Funktion die Zielgas-Konzentration als Funktion der Detektionsgröße (Q) und der Elektrolyt-Feuchte (Ef) beschreibt, und
das Analysegerät (100) dazu ausgestaltet ist,
bei der Messung der Zielgas-Konzentration die vorgegebene Konzentrations-Funktion auf die gemessene Detektionsgröße (Q) und die ermittelte Elektrolyt-Feuchte (Ef) anzuwenden.

3. Analysegerät (100) nach einem der vorhergehenden Ansprüche,
**dadurch gekennzeichnet, dass**

das Analysegerät (100) dazu ausgestaltet ist,
abhängig von der gemessenen Detektionsgröße (Q) des fließenden elektrischen Stroms (I) zu entscheiden, ob die Gasprobe (Pr) das Zielgas mit einer Konzentration innerhalb oder außerhalb eines vorgegebenen unteren Konzentrations-Wertebereichs aufweist oder nicht, und
mindestens dann, wenn die Zielgas-Konzentration außerhalb des Konzentrations-Wertebereichs liegt, die Elektrolyt-Feuchte (Ef) zu ermitteln.

4. Analysegerät (100) nach einem der vorhergehenden Ansprüche,
**dadurch gekennzeichnet, dass**

mindestens ein Verlaufs-Parameter des Stromstärken-Verlaufs [I(t)] die Zeitspanne [$T_{70}$, $T_{70,35}$, $T_{70,70}$, $T_{norm}$], die zwischen

- dem Beginn der Messung und
- dem frühesten oder spätesten Zeitpunkt ($t_{i,70}$), an dem der Stromstärken-Verlauf [I(t)] gleich $x_1$% der maximalen Stromstärke ($I_{max}$) wird,

verstreicht,
wobei $x_1$ und $x_2$ zwei Prozentwerte sind, die miteinander übereinstimmen oder voneinander verschieden sind, oder
die Zeitspanne, die zwischen

- dem frühesten oder spätesten Zeitpunkt ($t_{i,40}$, $t_{i,70}$), an dem der Stromstärken-Verlauf [I(t)] gleich $x_1$% der maximalen Stromstärke ($I_{max}$) wird, und
- dem frühesten oder spätesten Zeitpunkt ($t_{d,30}$, $t_{d,70}$), an dem der Stromstärken-Verlauf [I(t)] gleich $x_2$% der maximalen Stromstärke ($I_{max}$) wird,

verstreicht, oder
die elektrische Ladung ($Q_k$), die zwischen

- dem Beginn der Messung und
- dem frühesten oder spätesten Zeitpunkt ($t_{i,70}$), an dem der Stromstärken-Verlauf [I(t)] gleich $x_1$% der maximalen Stromstärke ($I_{max}$) wird,

fließt, oder
die elektrische Ladung ($Q_k$), die zwischen

- dem frühesten oder spätesten Zeitpunkt ($t_{i,40}$, $t_{i,70}$), an dem der Stromstärken-Verlauf [I(t)] gleich $x_1$% der maximalen Stromstärke ($I_{max}$) wird, und
- dem frühesten oder spätesten Zeitpunkt ($t_{d,30}$, $t_{d,70}$), an dem der Stromstärken-Verlauf [I(t)] gleich $x_2$% der maximalen Stromstärke ($I_{max}$) wird,

fließt, oder
die Steigung einer Sekante (Sk) zwischen

- dem Beginn der Messung und dem frühesten oder spätesten Zeitpunkt ($t_{i,70}$), an dem der Stromstärken-Verlauf [I(t)] gleich $x_1$% der maximalen Stromstärke ($I_{max}$) wird, oder
- dem frühesten oder spätesten Zeitpunkt ($t_{i,40}$, $t_{i,70}$), an dem der Stromstärken-Verlauf [I(t)] gleich $x_1$% der maximalen Stromstärke ($I_{max}$) wird, und dem frühesten oder spätesten Zeitpunkt ($t_{d,30}$, $t_{d,70}$), an dem der Stromstärken-Verlauf [I(t)] gleich $x_2$% der maximalen Stromstärke ($I_{max}$) wird.

5. Analysegerät (100) nach einem der vorhergehenden Ansprüche,
**dadurch gekennzeichnet, dass**

ein Verlaufs-Parameter des Stromstärken-Verlaufs [I(t)] die Zeitspanne ($T_{norm}$) zwischen den folgenden beiden Zeitpunkten ist:

- dem frühesten Zeitpunkt, an dem der Stromstärken-Verlauf [I(t)] gleich $x_1$% der maximalen Stromstärke ($I_{max}$) wird, und
- dem spätesten Zeitpunkt, an dem der Stromstärken-Verlauf [I(t)] gleich $x_2$% der maximalen Stromstärke ($I_{max}$) wird,
wobei $x_1$ und $x_2$ zwei Prozentwerte sind, die miteinander übereinstimmen oder voneinander verschieden sind,

wobei bevorzugt $x_1$ größer als $x_2$ ist,
wobei die Feuchte-Funktion ein Quotient aus einer Zähler-Feuchte-Funktion und einer Nenner-Feuchte-Funktion ist,
wobei in der Nenner-Feuchte-Funktion die Zeitspanne ($T_{norm}$) zwischen dem frühesten und dem spätesten Zeitpunkt auftritt und

wobei in der Zähler-Feuchte-Funktion mindestens ein anderer Verlaufs-Parameter [$T_{70}$, $T_{70,35}$, $T_{70,70}$] des Stromstärken-Verlaufs [I(t)] auftritt.

**6.** Analysegerät (100) nach Anspruch 5,
**dadurch gekennzeichnet, dass**

die Nenner-Feuchte-Funktion gleich der Zeitspanne ($T_{norm}$) zwischen dem frühesten und dem spätesten Zeitpunkt ist und

die Zähler-Feuchte-Funktion ein gewichtetes Mittel von mindestens zwei anderen Verlaufs-Parametern [$T_{70}$, $T_{70,35}$, $T_{70,70}$] des Stromstärken-Verlaufs [I(t)] ist.

**7.** Analysegerät (100) nach einem der vorhergehenden Ansprüche,
**dadurch gekennzeichnet, dass**

das Steuergerät (6) dazu ausgestaltet ist, ein Signal von einem Temperatursensor (52) zu empfangen,

wobei das Signal eine vom Temperatursensor (52) gemessene Temperatur in einer Umgebung des elektrochemischen Sensors (12) beschreibt und

wobei die vorgegebene Feuchte-Funktion die Feuchte (Ef) des Elektrolyten (28) als Funktion der vorgegebenen Verlaufs-Parameter [$T_{70}$, $T_{70,35}$, $T_{70,70}$, $T_{norm}$] und der Umgebungstemperatur beschreibt.

**8.** Mess-Verfahren zum Messen einer Konzentration eines Zielgases in einer Gasprobe (Pr)

unter Verwendung eines Analysegeräts (100),

wobei das Analysegerät (100)

- eine Messkammer (3),
- zwei Elektroden (20, 21),
- einen Stromstärken-Sensor (38) und
- einen ionisch leitenden Elektrolyten (28) zwischen den beiden Elektroden (20, 21)

umfasst,

wobei der Elektrolyt (28) eine zeitlich veränderliche Feuchte (Ef) aufweist,

wobei mehrere Verlaufs-Parameter [$T_{70}$, $T_{70,35}$, $T_{70,70}$, $T_{norm}$] und eine rechnerauswertbare Feuchte-Funktion vorgegeben werden,

wobei jeder vorgegebene Verlaufs-Parameter [$T_{70}$, $T_{70,35}$, $T_{70,70}$, $T_{norm}$] ein Parameter eines zeitlichen Verlaufs [I(t)] einer Stromstärke ist,

wobei die vorgegebene rechnerauswertbare Feuchte-Funktion die Feuchte (Ef) des Elektrolyten (28) als Funktion der vorgegebenen Verlaufs-Parameter [$T_{70}$, $T_{70,35}$, $T_{70,70}$, $T_{norm}$] beschreibt,

wobei das Mess-Verfahren die Schritte umfasst, dass

- ermöglicht oder bewirkt wird, dass die Gasprobe (Pr) in die Messkammer (3) fließt,
- das Zielgas als Bestandteil der Gasprobe (Pr) in der Messkammer (3) eine elektrochemische Reaktion nach Art einer Brennstoffzelle hervorruft,
- die bewirkte elektrochemische Reaktion das Fließen eines elektrischen Stroms (I) zwischen den beiden Elektroden (20, 21) bewirkt,
- der Stromstärken-Sensor (38) wiederholt die aktuelle Stärke fließenden elektrischen Stroms (I) misst,
- eine Detektionsgröße des fließenden elektrischen Stroms (I), insbesondere die elektrische Ladung (Q), gemessen wird und
- die Zielgas-Konzentration abhängig von der gemessenen Detektionsgröße (Q) des fließenden elektrischen Stroms (I) gemessen wird,

wobei das Mess-Verfahren die weiteren Schritte umfasst, dass

- ein tatsächlicher Stromstärken-Verlauf [I(t)] ermittelt wird, das ist der zeitliche Verlauf der Stärke (I) des Stroms, dessen Fließen durch die elektrochemische Reaktion hervorgerufen wird, und
- die aktuelle Feuchte (Ef) des Elektrolyten (28) unter Verwendung des ermittelten tatsächlichen Stromstärken-Verlaufs [I(t)] ermittelt wird,

wobei der Schritt, die aktuelle Elektrolyt-Feuchte (Ef) zu ermitteln, die Schritte umfasst, dass

- für jeden vorgegebenen Verlaufs-Parameter [$T_{70}$, $T_{70,35}$, $T_{70,70}$, $T_{norm}$] ermittelt wird, welchen Wert dieser Verlaufs-Parameter [$T_{70}$, $T_{70,35}$, $T_{70,70}$, $T_{norm}$] für den ermittelte Stromstärken-Verlauf [I(t)] annimmt, und
- die vorgegebene Feuchte-Funktion auf die ermittelten Verlaufs-Parameter-Werte angewendet wird.

**9.** Mess-Verfahren nach Anspruch 8,
**dadurch gekennzeichnet, dass**

in rechnerauswertbarer Form eine Konzentrations-Funktion vorgegeben wird,
wobei die Konzentrations-Funktion die Zielgas-Konzentration als Funktion der Detektionsgröße (Q) und der Elektrolyt-Feuchte (Ef) beschreibt, und
der Schritt, die Zielgas-Konzentration zu messen, den Schritt umfasst, dass
die vorgegebene Konzentrations-Funktion auf die gemessene Detektionsgröße (Q) und die ermittelte Elektrolyt-Feuchte (Ef) angewendet wird.

**10.** Mess-Verfahren nach Anspruch 8 oder Anspruch 9,
**dadurch gekennzeichnet, dass**

abhängig von der gemessenen Detektionsgröße (Q) des fließenden elektrischen Stroms (I) entschieden wird, ob die Gasprobe (Pr) das Zielgas mit einer Konzentration außerhalb eines vorgegebenen unteren Konzentrations-Wertebereichs aufweist oder nicht, und
die Detektion, dass die Gasprobe (Pr) das Zielgas mit einer Konzentration außerhalb des Konzentrations-Wertebereichs aufweist, den Schritt auslöst, dass die aktuelle Elektrolyt-Feuchte (Ef) ermittelt wird.

**11.** Mess-Verfahren nach einem der Ansprüche 8 bis 10,
**dadurch gekennzeichnet, dass**

mindestens ein Verlaufs-Parameter des Stromstärken-Verlaufs [I(t)]
die Zeitspanne [$T_{70}$, $T_{70,35}$, $T_{70,70}$, $T_{norm}$] zwischen den folgenden beiden Zeitpunkten ist:

- dem Beginn der Messung und dem frühesten Zeitpunkt ($t_{i,70}$), an dem der Stromstärken-Verlauf [I(t)] gleich $x_1$% der maximalen Stromstärke ($I_{max}$) wird, oder
- dem frühesten oder spätesten Zeitpunkt ($t_{i,40}$, $t_{i,70}$), an dem der Stromstärken-Verlauf [I(t)] gleich $x_1$% der maximalen Stromstärke ($I_{max}$) wird, und dem frühesten oder spätesten Zeitpunkt ($t_{d,30}$, $t_{d,70}$), an dem der Stromstärken-Verlauf [I(t)] gleich $x_2$% der maximalen Stromstärke ($I_{max}$) wird,
wobei $x_1$ und $x_2$ zwei Prozentwerte sind, die miteinander übereinstimmen oder voneinander verschieden sind, oder

die elektrische Ladung ($Q_k$), die zwischen

- dem frühesten oder spätesten Zeitpunkt ($t_{i,40}$, $t_{i,70}$), an dem der Stromstärken-Verlauf [I(t)] gleich $x_1$% der maximalen Stromstärke ($I_{max}$) wird, und
- dem frühesten oder spätesten Zeitpunkt ($t_{d,30}$, $t_{d,70}$), an dem der Stromstärken-Verlauf [I(t)] gleich $x_2$% der maximalen Stromstärke ($I_{max}$) wird,

fließt, oder
die Steigung einer Sekante (Sk) zwischen

- dem frühesten oder spätesten Zeitpunkt ($t_{i,40}$, $t_{i,70}$), an dem der Stromstärken-Verlauf [I(t)] gleich $x_1$% der maximalen Stromstärke ($I_{max}$) wird, und
- dem frühesten oder spätesten Zeitpunkt ($t_{d,30}$, $t_{d,70}$), an dem der Stromstärken-Verlauf [I(t)] gleich $x_2$% der maximalen Stromstärke ($I_{max}$) wird.

EP 4 592 672 A1

**FIG. 1**

**FIG. 2**

EP 4 592 672 A1

FIG. 3a

EP 4 592 672 A1

FIG. 3b

EP 4 592 672 A1

**FIG. 4**

FIG. 5

EP 4 592 672 A1

FIG. 6

Europäisches
Patentamt

European
Patent Office

Office européen
des brevets

# EUROPÄISCHER RECHERCHENBERICHT

**Nummer der Anmeldung**

EP 25 15 2014

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | US 2020/393405 A1 (SCHEFFLER TOWNER BENNETT [US]) 17. Dezember 2020 (2020-12-17) | 1-6,9-11 | INV. G01N27/413 G01N27/42 |
| Y | * Absätze [0002], [0006] - [0020], [0051] - [0081]; Abbildungen 1-7 * ----- | 7,12 | G01N33/497 |
| A | US 9 213 016 B1 (STETTER JOSEPH R [US] ET AL) 15. Dezember 2015 (2015-12-15) * das ganze Dokument * ----- | 1-13 | |
| Y | US 2003/183437 A1 (MENDOZA JOAQUIN L [US]) 2. Oktober 2003 (2003-10-02) * Spalte 7, Zeilen 52-67; Abbildung 14 * ----- | 7,12 | |
| A | US 2024/011939 A1 (BAESLER MALTE [DE] ET AL) 11. Januar 2024 (2024-01-11) * das ganze Dokument * ----- | 1-13 | |
| X | US 2020/225211 A1 (WILLKOMM WAYNE ROBERT [US] ET AL) 16. Juli 2020 (2020-07-16) * Absätze [0104] - [0105]; Abbildung 2 * ----- | 6,11 | |
| A | US 11 029 294 B2 (HONEYWELL INT INC [US]) 8. Juni 2021 (2021-06-08) * das ganze Dokument * ----- | 1-13 | RECHERCHIERTE SACHGEBIETE (IPC) G01N |
| A | US 11 112 378 B2 (MSA TECHNOLOGY LLC [US]) 7. September 2021 (2021-09-07) * das ganze Dokument * ----- | 1-13 | |

Der vorliegende Recherchenbericht wurde für alle Patentansprüche erstellt

| Recherchenort | Abschlußdatum der Recherche | Prüfer |
|---|---|---|
| München | 26. Mai 2025 | Lazar, Zala |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

**ANHANG ZUM EUROPÄISCHEN RECHERCHENBERICHT
ÜBER DIE EUROPÄISCHE PATENTANMELDUNG NR.**

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

26-05-2025

| Im Recherchenbericht angeführtes Patentdokument | Datum der Veröffentlichung | Mitglied(er) der Patentfamilie | Datum der Veröffentlichung |
|---|---|---|---|
| US 2020393405 A1 | 17-12-2020 | AU 2020290902 A1 | 27-01-2022 |
| | | CA 3143153 A1 | 17-12-2020 |
| | | CN 114207426 A | 18-03-2022 |
| | | EP 3983788 A1 | 20-04-2022 |
| | | JP 2022536720 A | 18-08-2022 |
| | | US 2020393405 A1 | 17-12-2020 |
| | | WO 2020251934 A1 | 17-12-2020 |
| US 9213016 B1 | 15-12-2015 | EP 3183566 A1 | 28-06-2017 |
| | | US 9213016 B1 | 15-12-2015 |
| | | WO 2016028983 A1 | 25-02-2016 |
| US 2003183437 A1 | 02-10-2003 | KEINE | |
| US 2024011939 A1 | 11-01-2024 | AU 2023204211 A1 | 25-01-2024 |
| | | DE 102022116825 A1 | 11-01-2024 |
| | | EP 4303577 A1 | 10-01-2024 |
| | | US 2024011939 A1 | 11-01-2024 |
| US 2020225211 A1 | 16-07-2020 | US 2020225211 A1 | 16-07-2020 |
| | | WO 2020146671 A1 | 16-07-2020 |
| | | ZA 202104677 B | 25-01-2023 |
| US 11029294 B2 | 08-06-2021 | CN 111796007 A | 20-10-2020 |
| | | CN 115389577 A | 25-11-2022 |
| | | EP 3719487 A1 | 07-10-2020 |
| | | EP 3901625 A1 | 27-10-2021 |
| | | US 2020319155 A1 | 08-10-2020 |
| | | US 2021262998 A1 | 26-08-2021 |
| US 11112378 B2 | 07-09-2021 | AU 2020290902 A1 | 27-01-2022 |
| | | CA 3143153 A1 | 17-12-2020 |
| | | CN 114207426 A | 18-03-2022 |
| | | EP 3983788 A1 | 20-04-2022 |
| | | JP 2022536720 A | 18-08-2022 |
| | | US 2020393405 A1 | 17-12-2020 |
| | | WO 2020251934 A1 | 17-12-2020 |

EPO FORM P0461

Für nähere Einzelheiten zu diesem Anhang : siehe Amtsblatt des Europäischen Patentamts, Nr.12/82